# EUROPEAN PATENT APPLICATION

(11) **EP 4 417 689 A1**
(43) Date of publication of application: **21.08.2024**
(21) Application number: 22880345.8
(22) Date of filing: 12.10.2022
(51) Int. Cl.: C12N 7/02, A61K 48/00, C12N 7/00

(54) **HEK293 CELL LINE ADAPTED TO SERUM-FREE SUSPENSION CULTURE AND USE THEREOF**

(30) Priority: 12.10.2021 CN 202111190063
(71) Applicant: Jiangsu Genscript Probio Biotech Co., Ltd., Zhenjiang, Jiangsu 212000 (CN)
(72) Inventor: WANG, Yan, Zhenjiang, Jiangsu 212000 (CN); PAN, Xiao, Zhenjiang, Jiangsu 212000 (CN); DING, Rongna, Zhenjiang, Jiangsu 212000 (CN); HUI, Erjing, Zhenjiang, Jiangsu 212000 (CN); XUAN, Chunling, Zhenjiang, Jiangsu 212000 (CN)
(74) Representative: Ström & Gulliksson AB
(86) International application number: PCT/CN2022/124873
(87) International publication number: WO 2023/061409

(57) **Abstract**

A HEK293 cell line adapted to serum-free suspension culture and a use thereof. Specifically, the present application relates to a method for preparing a viral vector such as an adeno-associated virus vector using the cell line, and a method for screening the cell line.

## Description

### Related Application and Incorporated by Reference

All documents cited or mentioned in the present application (including but not limited to all literatures, patents, and published patent applications cited herein) ("the documents cited in the present application"), all documents cited or mentioned in the documents cited in the present application, and the manufacturer's manuals, instructions, product specifications and product pages of any products mentioned in the present application or in any documents cited in the present application are incorporated by reference into the present application, may be adopted in the practice of the present invention. More specifically, all references are incorporated by reference into the present application as if each individual document is incorporated by reference. Any Genbank sequences mentioned herein are incorporated by reference into the present application. The present application claims priority to Chinese patent application No. 202111190063.4, filed on October 12, 2021, which is incorporated herein by reference in its entirety.

Citation of any document in the present application does not constitute an admission that such document is prior art to the present application.

### Field of the Invention

The present application relates to a method for screening a HEK293 cell strain adapted to serum-free suspension culture, and a HEK293 cell strain screened by using the method, especially PowerS^{™}-293 with a deposit number of CGMCC NO.: 23020. The present application further relates to a method for producing a viral vector, such as an adeno-associated virus vector, using the screened cell strain, especially PowerS^{™}-293.

### Background Art

Gene therapy and cell therapy have entered a stage of rapid development and become the most promising development direction of medical care for life. The main goal of gene therapy is to introduce an exogenous gene into a target cell or tissue, replace, compensate, block, and modify a specific gene to achieve the purpose of treating a disease. In gene therapy, 70%-80% of therapeutic regimens are accomplished by a viral vector. The viral vector is the key to delivering a therapeutic exogenous gene to a target site, and can be injected directly as a drug or used in drug production.

For many serious and life-threatening diseases, gene therapy is currently the most and only effective treatment, and at the same time, the indications for gene therapy are changing from rare/ultra-rare diseases to more common diseases. Therefore, larger-scale production of viral vectors for gene therapy is an urgent need for the industry.

Viral vectors used to introduce exogenous genes into target cells are mainly derived from viral vectors such as retroviruses, adenoviruses, and adeno-associated viruses (AAV). Among them, adeno-associated virus vectors are the most widely used. Currently, mainstream AAV production uses HEK293 cells as producer cells. HEK293 cells are cultured adherently by adding fetal bovine serum in systems such as flasks and Petri dishes, and the viruses are obtained by transient transfection with multiple plasmids and packaging. For the enterprises that research and develop cell and gene therapies, this production method of transiently transfecting adherent cells HEK293 is suitable for preclinical research, IND filing and clinical trials, because the demand for viral vectors is limited. This production method requires the use of fetal bovine serum, which is expensive and has potential risks of animal-derived virus contamination. If there is a need to increase cell production, it mainly relies on increasing the number of cell culture units, which often results in problems such as large space occupation and heavy workload. When larger-scale AAV production is required, one solution is to culture the producer cell strains in suspension. Compared with adherent culture, not only can suspension culture increase cell density, but also culturing cells in serum-free medium can reduce potential immunogenic substance contamination and animal-derived components in the final product, and simplify the downstream purification process, thereby greatly promoting the transformation of products to clinical grade. In addition, the scale-up of suspension culture can also reduce batch-to-batch variability compared with the original adherent culture by increasing the number of culture units.

As mentioned above, HEK293 cells are commonly used producer cells for AAV vectors, also known as human embryonic kidney cells 293 which is a cell line derived from human embryonic kidney cells and has the characteristics of high transfection efficiency and easy culture. During the suspension adaption process of HEK293 cells, many problems would arise. For example, (1) the current suspension adaption method mainly uses serum-reduced suspension adaption , and the adaption period is as long as usually about 3 months; (2) during the suspension adaption process, HEK293 cells are very prone to clumping, which would prevent many of the plasmids used for vector production from transfecting cells, and cells in the middle of large clumps die from lack of nutrients; currently, the main method for improving cell clumping is to add anti-clumping agents which would also significantly reduce the transfection efficiency; (3) the medium has a significant impact on cell growth status, cell density, and clumping; when HEK293 cells are adapted to different culture media, their growth performance are different; and (4) during the subcloning process, if the selected cell strain is not a monoclonal one, instability in virus expression quantity in different cell generations may occur during the later virus production test experiments.

In addition, although there are currently no exact regulations regarding monoclonal origin in gene therapy, monoclonal origin is one of the necessary conditions for product quality and expression stability. The current methods for screening monoclonal cells include (1) two rounds of limiting dilution to control the diluted cell density to be less than 0.5 cells/well; (2) one round of limiting dilution plus monoclonal imaging and photographing; (3) FACS plus one round of limiting dilution or monoclonal imaging and photographing; and (4) semi-solid culture. The existing problem is that the cell viability after FACS sorting is low, and it is not feasible to photograph HEK293 cells selected in semi-solid medium, and data on the monoclonal origin cannot be provided. The most commonly used method at present is one round of limiting dilution plus monoclonal imaging and photographing.

### Summary of the Invention

Through extensive experiments, the inventors of the present application found a method for screening a HEK293 cell strain adapted to serum-free suspension culture. By this method, a cell strain having high dispersibility and high proliferation density can be screened out.

Thus, in one aspect, the present application provides a method for screening a HEK293 cell strain adapted to serum-free suspension culture, comprising:
i) taking a HEK293 cell adherently cultured in a serum-containing medium, and placing the cell in a serum-free medium for suspension culture; and
ii) selecting a monoclonal cell strain adapted to serum-free suspension culture.

The serum-containing medium in step i) may contain 10% serum, such as 10% fetal bovine serum.

The suspension culture in step i) may include shaking culture at a condition of 37°C and 8% CO₂.

The suspension culture in step i) may include cell passaging.

The serum-free medium can be selected from OPM-293 CD03 medium (Shanghai OPM Biosciences Co., Ltd., 81070-001), LV-MAX production medium (Gibco, A35834-01), and Expi293 expression medium (Gibco, A14351-01). In one embodiment, the serum-free medium is OPM-293 CD03 medium.

Step ii) may include, after at least one round of limiting dilution and imaging and photographing, selecting monoclonal cells adapted to serum-free suspension culture, and culturing the selected monoclonal cells in suspension. In one embodiment, step ii) includes, after two rounds of limiting dilution and imaging and photographing, selecting monoclonal cells adapted to serum-free suspension culture, and culturing the selected monoclonal cells in suspension.

Step ii) may also include testing the viral vector production ability of the selected monoclonal cells, for example, detecting the titer of the viral vector produced.

Culturing the selected cells in suspension in step ii) may include cell passaging.

During the implementation of this method, no anti-clumping agent needs to be added.

In the method of the present application, 1) the cells adherently cultured in the serum-containing medium are directly subjected to serum-free suspension culture, and there is no step of progressive serum decreasing, which can greatly shorten the adaption and screening process, and the adaption and screening period can be shortened to about 20 days; 2) the serum-free medium can be used to culture the cells, avoid potential immunogenic substance contamination and animal-derived components, and simplify the downstream purification process; 3) the serum-free medium selected from LV-MAX production medium, Expi293 expression medium and OPM-293 CD03 medium, especially OPM-293 CD03 medium, is used, and is more conducive to screening the HEK293 cell strain suitable for suspension culture, that is, with high proliferation density and high dispersibility; 4) the use of the anti-clumping agent is avoided, and the transfection efficiency of the cells is not affected; and 5) at least one round, such as two rounds, of limiting dilution and imaging ensure the monoclonal origin, ensure that the cells provide stable virus expression, and also provide intuitive data support for the monoclonal origin.

The present application also encompasses the use of a medium selected from LV-MAX production medium (Gibco, A35834-01), Expi293 expression medium (Gibco, A14351-01) and OPM-293 CD03 medium (Shanghai OPM Biosciences Co., Ltd., 81070-001) in screening a HEK293 cell strain with high dispersibility and high proliferation density. In one embodiment, the screening medium is OPM-293 CD03 medium.

Since the growth status and clumping situation of cells in different media are not exactly the same, the inventors of the present application found that in OPM-293 CD03 medium, LV-MAX production medium and Expi293 expression medium, HEK293 cells can achieve a high density (about 1 × 10⁷ cells/ml) and a relatively low proportion of clumped cells. Among these three media, OPM-293 CD03 medium has the best screening effect.

In another aspect, the present application relates to the HEK293 cell strain screened by using the above method.

The HEK293 cell strain screened by the method of the present application can grow in suspension culture in a serum-free medium. The serum-free medium can be selected from LV-MAX production medium, Expi293 expression medium and OPM-293 CD03 medium. In one embodiment, the serum-free medium is OPM-293 CD03 medium.

Cell clumping can not appear during the suspension culture of the cell strain. In one embodiment, the cell strain does not clump in a liquid medium.

This cell strain can be used to produce viral vectors, especially adeno-associated virus vectors.

In particular, the present application provides a novel human embryonic kidney HEK293 cell strain PowerS^{™}-293, which was deposited at the China General Microbiological Culture Collection Center (CGMCC) on August 9, 2021 according to the Budapest Treaty and has a deposit number of CGMCC No.: 23020. PowerS^{™}-293 cell strain can grow in suspension culture in the serum-free medium, with high proliferation density, good viability, and no cell clumping, that is, with high dispersibility. Therefore, no additional anti-clumping agent is needed in the suspension culture process, and this cell strain can produce high titers of adeno-associated virus.

In yet another aspect, the present application relates to the use of the screened HEK293 cell strain in the preparation of a viral vector.

Specifically, the present application provides a method for preparing a viral vector using the cell strain screened by using the present application, including PowerS^{™}-293, comprising:
i) culturing the cell strain;
ii) introducing a viral vector expression system into the cell strain;
iii) culturing the cell strain under a condition favorable for the production of the viral vector; and
iv) collecting the viral vector.

Step i) may include suspension culture of the cell strain. In one embodiment, the cell strain is cultured with shaking in a serum-free medium under the condition of 37°C and 8% CO₂. The serum-free medium can be selected from LV-MAX production medium, Expi293 expression medium and OPM-293 CD03 medium. In one embodiment, the serum-free medium is OPM-293 CD03 medium.

Step i) may also include passaging of the cell strain.

Step ii) may include introducing the viral expression system into the cell strain via, for example, calcium phosphate co-precipitation and polyethylenimine (PEI)-mediated transfection. In one embodiment, the viral expression system is introduced into the cell strain via polyethylenimine (PEI)-mediated transfection.

The viral vector may be an adeno-associated virus vector. The adeno-associated virus vector can be adeno-associated virus vectors of all serotypes, and chimeras and hybrids thereof. In one embodiment, the adeno-associated virus vector is selected from AAV2, AAV5, AAV8, AAV9 and AAV10.

The viral vector expression system can include a heterologous nucleic acid-containing carrier plasmid, a rep-cap-containing packaging plasmid, and a helper plasmid. The helper plasmid can contain Ad5 genes, including VA, E2A and E4OEF6. The length of the heterologous nucleic acid in the carrier plasmid is no greater than about 4.7 kb.

The heterologous nucleic acid can encode a protein of interest or an untranslated RNA. In one embodiment, the heterologous nucleic acid contains an open reading frame encoding a protein of interest or an untranslated RNA. The heterologous nucleic acid can encode a polypeptide or a protein to achieve overexpression of the polypeptide or the protein. The heterologous nucleic acid can encode shRNA and interfere with the expression of a target gene. The heterologous nucleic acid can encode gRNA and Cas9 to achieve knockout of a target gene. The heterologous nucleic acid can encode gRNA and dCas9 to achieve endogenous overexpression of a target gene. The carrier plasmid can contain a promoter that directs expression of a heterologous gene, such as a promoter that enables cell/tissue-specific expression.

Step iii) may include suspension culture of the cell strain. In one embodiment, the cell strain is cultured in suspension in a serum-free medium. In one embodiment, the cell strain is cultured with shaking in a serum-free medium under the condition of 37°C and 8% CO₂. The serum-free medium can be selected from LV-MAX production medium, Expi293 expression medium and OPM-293 CD03 medium. In one embodiment, the serum-free medium is OPM-293 CD03 medium.

Step iii) may include passaging the cells.

In step iv), the viral vector can be collected by lysing the cells, or the viral vector secreted by the cell strain can be collected from the medium.

The method can also include titer determination, purification, *etc.* of the viral vector after step iv).

Other features and advantages of the present disclosure will be apparent from the following detailed descriptions and examples, which should not be viewed as limiting. All citations, GenBank accession numbers, patents and published patent applications cited in the present application are expressly incorporated herein by reference.

It should be noted that in the present application, especially in the claims, terms such as "comprise", "include", *etc.,* terms such as "essentially consist of" allow the presence of elements not explicitly stated. The aspects and embodiments of the present invention described herein include aspects and embodiments involving "comprise", "consist of", and "essentially consist of".

As used in the description and appended claims of the present application, the singular forms "a," "an," and "the" include plural referents, unless the context clearly dictates otherwise. Therefore, for example, reference to "a molecule" optionally includes a combination of two or more of such molecules, and the like.

As used the present application, the term "about" refers to a conventional error range of the corresponding numerical value easily known by those skilled in the art. Reference herein to "about" a certain value or a parameter includes (and describes) the embodiment involving the value or parameter itself.

### Brief Description of the Drawings

The following detailed description is given by way of example and is not intended to limit the present invention to the specific embodiments, and can be better understood in conjunction with the accompanying drawings.
Fig. 1 shows the growth curve of adherent HEK293 cells.
Fig. 2 shows viral titers of AAV2, AAV5, AAV8 and AAV9 packaged in adherent HEK293 cells.
Fig. 3 shows morphological diagrams of cells adapted in different serum-free media.
Fig. 4 shows the growth curves of cells adapted in three serum-free media.
Fig. 5 shows screening images of monoclonal origin.
Fig. 6 shows a morphological diagram of monoclonal cells.
Fig. 7 shows the cell growth/viability curves of clone 13, clone 20 and clone 29.
Fig. 8 shows a graph showing AAV2 virus titer yields of clone 13, clone 20 and clone 29.
Fig. 9 shows a graph showing AA5, AAV8 and AAV9 virus titer yields of clone 13, clone 20 and clone 29.
Fig. 10 shows a graph showing AAV5 virus titer yields at passages 5, 10, 15 and 20 of clone 13.

Deposit instruction: The novel human embryonic kidney HEK293 cell strain PowerS-293 involved in the present application was deposited on August 9, 2021 in the China General Microbiological Culture Collection Center according to the Budapest Treaty, address: Institute of Microbiology, Chinese Academy of Sciences, No. 3, Yard 1, Beichen West Road, Chaoyang District, Beijing, China.
with a deposit number of CGMCC No.: 23020.

### Detailed Description of Embodiments

Unless defined otherwise, the technical and scientific terms used herein have the same meaning as commonly understood by a person skilled in the art to which the present invention belongs.

The present application provides a method for screening a cell strain adapted to serum-free suspension culture, and the screened cell strain. The cell strain has high dispersibility and high proliferation density, and can be used to produce a viral particle vector with high-titer, especially an adeno-associated virus vector particle.

### Viral vector

The viral vector is a tool used by molecular biologists to deliver a genetic material to a cell, carries an exogenous gene and can be packaged into a virus particle to mediate the transfer and expression of the exogenous gene, and are not pathogenic to the body. Known viral vectors include retroviral vectors, adenoviral vectors, adeno-associated virus (AAV) vectors, herpes virus, *etc.*

The terms "viral vector", "viral particle" or "viral vector particle" herein are used interchangeably herein. The term "heterologous nucleic acid", "heterologous gene", "exogenous nucleic acid" or "exogenous gene" refers to a nucleic acid or gene that does not naturally occur in a virus. Generally, the heterologous nucleic acid or exogenous gene contains an open reading frame encoding a protein of interest or an untranslated RNA.

### Adeno-associated virus

Adeno-associated virus (AAV) is a member of the Parvoviridae family, and is a type of icosahedral parvovirus that cannot replicate autonomously and has no envelope and contains a linear single-stranded DNA genome of approximately 4.7 kb.

The AAV genome consists of two open reading frames, Rep and Cap, flanked by two 145 bp inverted terminal repeats (ITRs). The ITRs pair to form a hairpin structure that enables primase-independent synthesis of the second complementary DNA strand. Rep and Cap can translate a variety of different proteins, including Rep78, Rep68, Rep52 and Rep40 required for the AAV life cycle, as well as capsid proteins VP1, VP2 and VP3.

"Adeno-associated virus" or "AAV" as used herein includes, but is not limited to, AAV type 1, AAV type 2, AAV type 3 (including types 3A and 3B), AAV type 4, AAV type 5, AAV type 6, AAV type 7, AAV type 8, AAV type 9, AAV type 10, AAV type 11, AAV type 12, AAV type 13, snake AAV, avian AAV, bovine AAV, canine AAV, equine AAV, sheep AAV, goat AAV, shrimp AAV and any other currently known or later discovered AAVs. The genomic sequences of various AAV serotypes, as well as the sequences of native ITRs, Rep proteins and capsid subunits, are known in the art. Such sequences can be found in the literature or in public databases such as GenBank.

The term "terminal repeat" or "TR" as used herein includes any viral terminal repeat sequence or synthetic sequence that forms a hairpin structure and acts as an inverted terminal repeat (ITR), *i.e.*, mediates a desired function, such as replication, viral packaging, integration and/or provirus rescue, and the like. The ITR can be an AAV ITR or a non-AAV ITR. Non-AAV ITR sequences are such as those from other parvoviruses (e.g., canine parvovirus, bovine parvovirus, mouse parvovirus, porcine parvovirus, and human parvovirus B-19), or the SV40 hairpin that serves as the SV40 origin of replication can be used as ITR, which can be further modified by truncation, substitution, deletion, insertion and/or addition. Further, ITRs can be partially or completely synthetic.

The AAV genome has palindromic sequences at its 5' and 3' ends. The palindromic nature of the sequence results in the formation of a hairpin structure that are stabilized by hydrogen bonds formation between complementary base pairs. The hairpin structure is thought to take a "Y" or "T" shape. "AAV inverted terminal repeat" or "AAV ITR" can be from any AAV, including but not limited to serotypes 1, 2, 3a, 3b, 4, 5, 6, 7, 8, 9, 10, 11 or 13, snake AAV, avian AAV, bovine AAV, canine AAV, equine AAV, sheep AAV, goat AAV, shrimp AAV and any other currently known or later discovered AAVs. The AAV ITR need not have native terminal repeat sequences, for example, the native AAV ITR sequence can be altered by insertions, deletions, truncations, and/or missense mutations, as long as the terminal repeats mediate the desired function, such as replication, viral packaging, integration, and/or protovirus rescue, and the like.

As one of the most commonly used viral vectors for gene therapy, AAV has the following characteristics.
1. AAV is highly safe and has low immunogenicity. AAV is a replication-deficient DNA virus with no autonomous replication ability. Wild-type AAV relies on the rep gene for low-frequency site-specific integration. There are currently no reports of human or mammalian diseases caused by AAV, and it is also one of the safest viral vectors among the gene therapy drugs approved by the FDA.
2. AAV has a broad host range and is capable of infecting both dividing and non-dividing cells.
3. AAV has strong diffusion ability. AAV has a diameter of about 20-26 nm, small size, high titer, and good diffusion ability. Some AAVs, such as AAV9, have the ability to cross the blood-brain barrier and are widely used in the field of neuroscience.
4. AAV expresses a gene *in vivo* for a long term. AAV has the ability to maintain long-term gene transcription expression. *In vivo* expression generally reaches a peak in 3 weeks, and then the high expression continues, with an effect time of more than 5 months.
5. There are various AAV serotypes. AAV can be divided into different serotypes based on the cellular receptor to which the capsid protein binds. There are 13 different serotypes of AAV in primates (*i.e.,* AAV1-AAV13), of which AAV2, AAV3, and AAV9 are derived from humans. Based on this characteristic of AAV, different serotypes of AAV can be selected to transfect different target tissue types. For the central nervous system, AAV1, AAV2, AAV4, AAV5, AAV8 and AAV9 are preferably used; For the heart, AAV1, AAV8 and AAV9 are preferably used; For the kidneys, AAV2 is preferably used; For the liver, AAV7, AAV8 and AAV9 are preferably used; For the lungs, AAV4, AAV5, AAV6 and AAV9 are preferably used; For the pancreas; AAV8 is preferred; For the photoreceptor cells, AAV2, AAV5 and AAV8 are preferably used; For the retinal pigment epithelial cells, AAV1, AAV2, AAV4, AAV5 and AAV8 are preferably used; For the skeletal muscle, AAV1, AAV6, AAV7, AAV8 and AAV9 are preferably used.

Serotype 2, *i.e,* AAV2, is the most studied and used AAV. AAV2 has been found to bind to 3 cell receptors, *i.e,* heparan sulfate proteoglycan (HSPG), integrin aVβ5, and fibroblast growth factor receptor 1 (FGFR-1). HSPG is the main receptor, and the other two are co-receptors, allowing AAV2 to enter cells through receptor-mediated endocytosis. Studies have found that AAV2 can kill cancer cells without harming normal cells.

Other serotypes have higher gene delivery efficiency than AAV2. As mentioned above, AAV6 performs better at infecting airway epithelial cells; AAV7 has a high transfection efficiency into skeletal muscle cells, similar to AAV1 and AAV5; AAV8 is good at transfecting cardiomyocytes; AAV1 and AAV5 are very efficient in gene delivery to vascular endothelial cells; Most serotypes can transfect neural cells, and AAV5 can also transfect astrocytes.

### Construction of adeno-associated virus vector

When constructing AAV carrier plasmids, since the ITR is the only cis-acting element required for genome replication and packaging, the rep and cap genes can be removed and cloned into separate plasmids. The promoter and the heterologous gene driven by the promoter can be cloned between two ITRs, allowing efficient packaging into AAV capsids.

AAV lacks the ability to replicate and therefore requires co-infection with helper viruses such as adenovirus (Ad) or herpes simplex virus (HSV). After co-infection with Ad or HSV, AAV utilizes several helper viral early genes to promote its replication. Infection of Ad into producer cells to produce AAV is efficient in producing AAV, but the consequence is the production of an excessive quantity of Ad particles. Complete removal of Ad has relied on physical techniques such as CsCl gradients, column chromatography and thermal denaturation steps to inactivate any residual Ad particles that may still be present. Although most of these operations have been successful to varying degrees, the possibility of Ad contamination represents an unnecessary risk, and the presence of denatured Ad proteins is unacceptable for clinical use.

A significant improvement in the development of AAV production was the introduction of triple plasmid transfection. This method uses rep and cap plasmids and variants of ITR plasmids, avoiding the use of Ad. Specifically, the Ad proteins of E1A, E1B, E4, and E2A, as well as VA RNA, were cloned into a single plasmid called XX680. The Ad helper gene is supplied on the XX680 plasmid, thereby obtaining only the AAV vector in the transfected cells and avoiding the production of Ad. In preparation for transfection using HEK293 cells, the helper plasmid was further simplified to contain only VA, E2A, and E4OEF6 because HEK293 cells constitutively express E1A/1B. The carrier plasmid containing ITR and heterologous nucleic acid, the packaging plasmid containing rep-cap, and the helper plasmid containing VA, E2A and E4OEF6 constitute the AAV vector expression system.

As used herein, "AAV expression system" refers to a system of one or more polynucleotides that, when introduced into a suitable host cell, is sufficient to support the production of AAV.

As used herein, the "Rep gene" or "Rep coding sequence" of AAV refers to a nucleic acid sequence encoding a non-structural protein of AAV that mediates viral replication and the production of new viral particles. AAV replication genes and proteins have been described, for example, in Fields et al. Virology, Volume 2, Chapters 69 and 70 (4th ed., Lippincott-Raven Publishers). The "Rep coding sequence" need not encode all AAV Rep proteins. For example, in the case of AAV, the Rep coding sequence need not encode all four AAV Rep proteins (Rep78, Rep68, Rep52, and Rep40). The Rep coding sequence encodes at least those replication proteins required for viral genome replication and packaging into new virions. The Rep coding sequence would generally encode at least one large Rep protein (*i.e.* Rep78/68) and one small Rep protein (*i.e.* Rep52/40). In certain embodiments, the Rep coding sequence encodes AAV Rep78 protein and AAV Rep52 and/or Rep40 proteins. In other embodiments, the Rep coding sequence encodes Rep68 and Rep52 and/or Rep40 proteins. In yet further embodiments, the Rep coding sequence encodes Rep68 and Rep52 proteins, Rep68 and Rep40 proteins, Rep78 and Rep52 proteins, or Rep78 and Rep40 proteins. As used herein, the term "large Rep protein" refers to Rep68 and/or Rep78. The large Rep protein can be wild-type or synthetic. The wild-type large Rep protein can be derived from any AAV, including but not limited to serotypes 1, 2, 3a, 3b, 4, 5, 6, 7, 8, 9, 10, 11 or 13 and any others currently known or later discovered AAVs. The synthetic large Rep protein can be altered through insertions, deletions, truncation and/or missense mutations.

As used herein, an AAV "cap gene" or "cap coding sequence" encodes a structural protein that forms a functional AAV capsid (*i.e.,* that can package DNA and infect a target cell). Typically, the cap coding sequence would encode all AAV capsid subunits, but may encode less than all capsid subunits as long as a functional capsid is produced. Typically, the cap coding sequence would be present on a single nucleic acid molecule. The capsid structure of AAV is described in more detail in BERNARD N. FIELDS et al., VIROLOGY, Volume 2, Chapters 69 and 70 (4th ed., Publisher Lippincott-Raven).

A "carrier plasmid" as used herein generally requires only the ITR in *cis* to generate the virus, and all other viral sequences are optional and can be provided in *trans.* Typically, AAV carrier plasmids would retain only one or more ITR sequences in order to maximize the size of the heterologous gene that can be efficiently packaged by the vector. Structural and non-structural protein coding sequences can be provided in *trans* (for example, from a vector such as a plasmid, or by stable integration of the sequence into a packaging cell). In embodiments of the present application, the AAV carrier plasmid contains at least one ITR sequence (e.g., an AAV ITR sequence), optionally two ITRs (e.g., two AAV ITRs), which would typically be at the 5' and 3' ends of the vector genome and flanked by, but need not be adjacent to, a heterologous nucleic acid. ITRs can be the same or different from each other.

As research continues to deepen, it has been discovered that AAVs of different serotypes can hybridize, and the hybridized AAVs would have characteristics from both parent AAVs, thus giving rise to AAV subtypes. The generation of subtypes is usually achieved by combining the genome of one serotype with the capsid protein of another serotype. The AAV commonly used in research now is a hybrid viral vector produced by combining the AAV2 genome with different capsid proteins, generally labeled as AAV2/N (N represents different capsid serotypes). The recombinant virus has the stable expression and gene integration capabilities of AAV2, and at the same time has acquired tissue infection tropism of different serotypes, showing a certain organ targeting specificity. For example, AAV2/1 has tissue tropism for the nervous system, muscle, skeletal muscle, cardiac muscle, and smooth muscle; AAV2/2 has tissue tropism for retina, nervous system, muscle, liver and vascular smooth muscle; AAV2/3 has tissue tropism for muscle, liver, lungs and eyes; AAV2/4 has tissue tropism for nervous system, muscles, eyes and brain; AAV2/5 has tissue tropism for nervous system, lungs, retina, liver and synovial joints; AAV2/6 has tissue tropism for nervous system, lungs, muscle and heart; AAV2/7 has tissue tropism for muscle and liver; rAAV2/8 has tissue tropism for nervous system, liver, muscle, adipose tissue, pancreas and retina; AAV2/9 has tissue tropism for nervous system, myocardium, lung, retina, and skin; AAV-PHP.eB can cross the blood-brain barrier; AAV-PHP.S can target all peripheral nerves; *etc.* Studies have shown that compared to AAV2, AAV2/5 can infect more brain cell types and has a higher infection accuracy. AAV-DJ has hybrid capsid proteins from 8 different AAVs and can infect cells in many areas of the body. AAV can also modified in terms of surface amino acid residues to further evade recognition by the immune system.

### Preparation of viral vectors

The preparation of viral vector particles usually requires the following steps:
1) culturing and proliferating a host cell; 2) introducing a viral vector expression system into the host cell; 3) culturing and proliferating the host cell into which the viral vector expression system has been introduced; 4) collecting and purifying the viral vector produced.

Although it is now possible to package some viral vectors (such as AAV vectors) *in vitro* (cell-free), this packaging system still requires cell extracts, and the packaging efficiency is quite low, far from the level of production. So far, the packaging of viral vectors has mainly been carried out in host cells susceptible to the virus. Host cells not only provide the environmental conditions for virus replication and packaging, but also have many cellular components directly involved in the process of virus replication and packaging.

There are two types of host cells for virus production, one is a cell line that stably expresses elements of the viral packaging system, and the other is a transiently transfected cell line. The packaging method of transient transfection is easy to operate and saves time, and is the most commonly used method for producing viral vectors.

Host cells that stably express viral packaging elements are also called producer/packaging cell lines. In the context of adeno-associated vectors, the term "packaging cell line" may refer to a cell line with rep and cap genes introduced; "Producer cell line" may refer to a cell line with rep and cap genes, ITR and a foreign gene introduced. The packaging cell line requires co-transfection of the AAV carrier plasmid and the helper plasmid to prepare AAV, while the producer cell line only requires transfection of the helper plasmid to prepare AAV. In one embodiment, the "packaging cell line" is a cell line in which rep and cap genes are inserted into the cell genome; The "producer cell line" is a cell line in which rep and cap genes, ITRs, and exogenous genes are inserted into the cell genome. In another embodiment, the "packaging cell line" is a cell line in which rep and cap genes are stably inserted into the cell genome; The "producer cell line" is a cell line in which rep and cap genes, ITRs, and exogenous genes are stably inserted into the cell genome. The cell line stably transfected with AAV is usually Hela cells.

For transient transfection preparation of A VV, HEK293 cells are generally used. HEK293 cells are also known as human embryonic kidney cells 293 cells. Such cells constitutively express E1a/b, *i.e.,* one of the functional adenovirus genes required for AAV replication.

The first step in viral vector particle preparation is culturing and proliferating host cells. The culture of host cells is generally divided into adherent culture and suspension culture. To increase the production of viral vectors, the total surface area available for cell attachment can be increased, or cell suspension culture can be carried out.

As the most commonly used host cells for AAV production, HEK293 cells are usually adherently cultured in a cell culture flask containing 10% fetal bovine serum medium, and the virus yield is small. To increase the surface area to which cells can attach, multilayer containers such as HYPERFlask^{™} (Corning), and multilayer cell factories such as the cell factory system (Numc, EasyFill, ThermoScientific, Waltham, MA, USA) can be used. HYPERFlask^{™} (Corning) has a total growth area of approximately 1720 cm² and has a gas permeable surface for oxygen and carbon dioxide exchange, and the yield is about 10 times that of T-150 culture flask. Cell factory systems (Numc, EasyFill, ThermoScientific, Waltham, MA, USA) can have up to 40 layers, which can produce a culture surface area equivalent to that of about 170 T-150 culture flasks. In addition, fixed-bed bioreactors such as iCELLis (Pall Life Sciences, Hoegaarden, Belgium) and the Scale-X^{™} bioreactor system (Univercells, Gosselies, Belgium) have been developed on the market. The fixed-bed is filled with polyethylene terephthalate (PET) fibers or of PET fiber layers, providing a large surface area for cell attachment. The cell growth area provided by iCELLis 500 is equivalent to about 3000 HYPERFlasks.

Although there are various equipment updates that can expand the culture of serum-dependent adherent HEK293, it is still time-consuming and labor-intensive. Therefore, researchers began to try to adapt HEK293 cells to suspension culture. HEK293E cells are first adapted and cultured in suspension in serum-free medium. Since then, new such cell lines have been continuously developed, such as HEK293SF-3F6, *etc.* (Ansorge,S. et al., (2009) Development of a Scalable Process for High-Yield Lentiviral Vector Production by Transient Transfection of HEK293 Suspension Cultures. J. Gene Med. 11:868-876). Compared with adherent culture, suspension culture has many advantages, such as no need for complicated culture equipment, no need for mechanical force or enzymes to detach cells, more convenient for passaging, and convenient for scale-up.

For the enterprises that research and develop cell and gene therapies, the demand for viral vectors is limited during preclinical research, IND declaration and clinical trials, so almost all of the enterprises use transient transfection of adherent HEK293 cells to produce adeno-associated virus vectors. The adherent culture of HEK293 cells is usually in the form of a cell factory, which mainly relies on increasing the number of culture units to increase the number of cells. Therefore, when a large number of viral vectors is required, it often needs to occupy a large space, and the production labor is also large. To obtain large growth areas while reducing labor, one approach is to adapt adeno-associated virus-producing cell strains to suspension culture. Compared with adherent culture, suspension culture can greatly increase cell density.

The second step in the preparation of viral vector particles is to introduce the viral vector expression system into the host cell, that is, to transfect the host cell with a packaging nucleic acid vector, a helper nucleic acid vector, a nucleic acid vector carrying a heterologous gene, *etc.*

"Viral expression system" in the present application refers to a system of one or more polynucleotides which, when introduced into a suitable host cell, is sufficient to support viral replication and packaging. The viral expression system can also contain heterologous genes encoding nucleic acids such as RNA or polypeptides/proteins that can be packaged together into viral vector particles. An AAV expression system typically includes a polynucleotide encoding AAV rep and cap, a helper gene, and the rAAV genome (ITR, *etc.).* Herein, "nucleic acid vector" refers to a DNA or RNA vector, which may be a plasmid, or a bacterial artificial chromosome (BAC), a yeast artificial chromosome (YAC), a P1-derived artificial chromosome (PAC), fosmid or cosmid *etc.* in a specific embodiment. The nucleic acid vector may contain an origin of replication, a promoter, a transcriptional regulatory element, and the like as necessary. Any element can be operably linked to a promoter so that the expression can be controlled. The promoter mentioned herein may include known promoters (complete or partial), which may be constitutively active or inducible, for example, in the presence of a regulatory protein. In one embodiment, the nucleic acid vector additionally comprises a high-efficiency promoter, such as a CMV promoter. This promoter has the advantage of promoting high level expression of an element encoded on a non-mammalian nucleic acid vector. In another embodiment, the CMV promoter comprises a sequence derived from the human cytomegalovirus strain AD169. The sequence is available at Genome Accession No. X17403, for example from base pairs 173731 to 174404. In the present application, the nucleic acid vector carrying the exogenous gene may be called a carrier plasmid. The carrier plasmid typically contains a promoter (such as CMV), a 3' ITR, a 5' ITR, and a potential exogenous gene linked to the promoter.

"Transfection" or "infection" as described herein refers to the introduction of an exogenous genetic material, such as the "viral expression system" described herein, into a host cell. Commonly used transfection methods known to those skilled in the art include, but are not limited to, the use of physical methods (for example, electroporation, cell extrusion, sonoporation, optical transfection, protoplast fusion, impact transfection, magnetofection, genetic gun or particle bombardment), chemical reagents (for example, calcium phosphate, highly branched organic compounds, or cationic polymers), or cationic lipids (for example, lipofection). Many transfection methods require contacting cells with a solution of plasmid DNA, and the cells grow and are selected for expression of a mark gene.

It is not easy to introduce an exogenous nucleic acid or a nucleic acid vector into a host cell, it is necessary to cross the barrier of cell membrane, and high transfection efficiency is necessary to produce a viral vector particle with high titer. The method of viral plasmid transfection can be roughly divided into two kinds, chemical methods and physical methods. Commonly used chemical methods include calcium phosphate co-precipitation, polyethyleneimine (PEI)-mediated transfection, Lipofectamine (Invitrogen)-mediated transfection, *etc.,* and the physical methods include electroporation, such as flow electroporation, microinjection, gene gun, *etc.*

Calcium phosphate co-precipitation is suitable for a variety of cell types, enables a large number of cells to be transfected at the same time, and can be used for large-scale virus preparation. However, calcium phosphate is very sensitive to pH changes and is quite toxic to cells. To avoid toxicity, host cells need to be cultured in a medium containing serum or albumin, and the medium should be replaced in time after transfection.

PEI-mediated transfection is as efficient as calcium phosphate. When using this method, it is necessary to select an appropriate PEI/DNA ratio. PEI is also toxic to cells, but it is also possible to leave the medium unchanged after transfection. Furthermore, the PEI method is not sensitive to pH. The PEI method can be used for both adherent and suspension cultured cells, and serum in the medium is essential (Toledo, J. R. et al., (2009) Polyethylenimine-Based Transfection Method as a Simple and Effective Way to Produce Recombinant Lentiviral Vectors. Appl. Biochem. Biotechnol. 157: 538-544).

Lipofectamine-mediated transfection is the most efficient and convenient and suitable for a variety of cell types, and the transfection rate is very high, for example, 100% of the cells can be transfected with adeno-associated virus in some instances. However, the use of Lipofectamine is cost-prohibitive for large-scale viral vector preparation.

Flow electroporation is as efficient as the calcium phosphate method, but requires only one-third or less of the amount of DNA, is not toxic to cells, and is suitable for cells in suspension culture.

In addition, addition of sodium butyrate to the medium after transfection has been reported to increase virus titers.

Among the above transfection methods, calcium sulfate co-precipitation is more suitable for adherent cultured cells such as HEK293 cells, flow electroporation is more suitable for suspension cultured cells, and Lipofectamine is more suitable for small-scale applications due to cost reasons. In the present application, PEI was selected to mediate the transfection of HEK293 cells with nucleic acid vectors.

Once inside the host cell, some nucleic acid vectors would be randomly integrated into the endogenous genome of the mammalian host cell. Therefore, it may also be desirable to select a host cell into which the nucleic acid encoded on the nucleic acid vector has been integrated, for example, using an antibiotic resistance selectable marker such as a zeocin resistance marker, *etc.* Those skilled in the art would be aware of a method to facilitate integration of a nucleic acid vector, for example, linearizing the nucleic acid vector, if the nucleic acid vector, such as a plasmid, is naturally circular. The nucleic acid vector may additionally comprise regions having shared homology with the endogenous chromosome of the host cell to direct integration into a selected site in the endogenous genome. In addition, if recombination sites are present on the nucleic acid vector, they can be used for targeted recombination. Other methods of targeted integration are well known in the art. For example, methods that induce targeted cleavage of genomic DNA can be used to promote targeted recombination at selected chromosomal loci. These methods typically involve the use of an engineered cleavage system to induce double-strand breaks (DSBs) or nicks in the endogenous genome to repair the break by natural processes (such as non-homologous end joining) or to repair the break using a repair template (i.e., homology directional restoration or HDR). By using specific nucleases such as engineered zinc finger nucleases (ZFNs) and transcriptional activators such as effector nucleases (TALENs), using the CRISPR/Cas9 system and engineered crRNA/tracr RNA ('single guide RNA') to direct specific cleavage and/or use nucleases based on the Argonaute system (for example from *T. thermophilus,* called 'TtAgo', Swarts DC et al., (2014) DNA-guided DNA interference by aprokaryotic Argonaute. Nature. 507 (7491): 258-261), cleavage can occur. Targeted cleavage using one of these nuclease systems can utilize HDR or NHEJ-mediated processes to insert a nucleic acid at a specific target location. Therefore, in one embodiment, the nucleic acid sequence encoded on the nucleic acid vector is integrated into the genome (*i.e.,* the endogenous chromosome) of the host cell using at least one nuclease, wherein the at least one nuclease cleaves the genome of the host cell, allowing the integration of the nucleic acid sequence into the genome of the cell. In another embodiment, the at least one nuclease is selected from zinc finger nuclease (ZFN), TALE nuclease (TALEN), CRISPR/Cas nuclease system and a combination thereof.

Transfection is divided into transient transfection and stable transfection. In the case of transient transfection, the exogenous gene is expressed but not integrated into the genome of the cell and therefore not replicated. Transiently expressed exogenous genes in cells are expressed for a limited period of time, usually only for days or months, until the exogenous genes are lost due to various reasons during cell division. Stable transfection is based on transient transfection, but requires an important accidental process, that is, in a small number of transfected cells, the exogenous gene can be integrated into the genome of the cell. A hallmark of a stably transfected cell is that the exogenous gene becomes part of the genome of the cell and thus replicates. Progeny cells of stably transfected cells also express the exogenous gene, thus forming a stable cell line.

Transient transfection generally lasts for several days. Transient transfection is used to study gene expression. Cells are usually harvested within 24 h to 96 h after transfection, and the specific time depends on various factors such as cell type and vector construction. For this reason, transient transfection is generally used to study short-term expression of a gene or a gene product, gene knockout or RNA-mediated gene silencing, and small-scale protein synthesis. Transient mRNA transfection yields results faster than traditional plasmid DNA transfection because mRNA can be expressed directly outside the nucleus, and in some systems mRNA can be expressed within minutes after transfection. Correspondingly, if long-term gene expression is required, stable transfection must be performed, such as in the case of large-scale protein synthesis, long-term pharmacological research, gene therapy research, long-term genetic regulatory mechanism research, *etc.* Stable transfection takes longer and is more laborious than transient transfection.

The cell line stably transfected with AAV is usually Hela cells, such as HelaS3 cell line, which can have rep and cap genes, or rep and cap encoding genes introduced, and produce AAV under the condition of co-transfection of the AAV carrier plasmid and the helper plasmid; and which can also have rep and cap genes, ITRs and exogenous genes introduced, and produce AAV under the condition of transfection of the helper plasmid.

Since it is difficult to obtain stable transfected cell lines, the current mainstream virus preparation, such as adeno-associated virus vector preparation, still uses transient transfection of the HEK293 cell line in suspension culture.

The third step of viral vector preparation is to culture and proliferate the host cells into which the viral vector expression system has been introduced under conditions that produce viral vector particles, similar to the first step.

The fourth step is to isolate and purify the viral vector.

In one embodiment, the viral vector can be collected by lysing the cells, for example by allowing the cells to precipitate after removing the cells from the medium. In another embodiment, the viral vector can be collected from the cell medium, for example by isolating the vector secreted by the cells. Some or all of the medium may be collected from the culture at one time or more than once to collect AAV, for example, the medium is collected periodically during the culture steps (e.g., every 12, 18, 24, or 36 h), or for example, the medium is collected starting at about 48 h after transfection. After the medium is collected, fresh medium with or without additional nutritional supplements can be added to the culture. In one embodiment, cells can be cultured in a perfusion system, so that the medium flows constantly on the cells and is collected for isolating the secreted AAV. AAV can be collected from the medium continuously, for example, 48, 72, 96, or 120 h or longer after transfection, as long as the transfected cells remain viable. In certain embodiments, certain secreted AAV serotypes, such as AAV8 and AAV9, that do not bind to the host cells or only loosely bind to the host cells are collected from the medium.

The collected cell medium containing viral vector particles may contain a variety of impurities, such as host cells, other proteins produced by host cells, plasmid DNA, serum, *etc.* The serum components are complex and may contain endotoxin, immunogenic protein, *etc.* These impurities need to be removed and the final product need to be concentrated. In these treatments, the biggest problem is how to maintain the activity/functionality of the viral vector, therefore, it is necessary to use as few steps as possible to complete the treatments in period of time as short as possible. The methods used for small-scale purification are obviously not suitable for large-scale production. Moreover, some purification steps, such as centrifugation, do not comply with cGMP regulations, and highspeed centrifugation would destroy the viral envelope to a certain extent.

Existing methods for large-scale purification of viral vectors include clarification, concentration, and purification.

Clarification refers to the removal of host cells and host cell debris after collection of the supernatant. Small-scale clarification can be carried out by methods such as centrifugation and microfiltration, while large-scale clarification preferably uses 45 µm porous membranes. To avoid pore clogging, membranes with decreasing pore size can be used to improve filtration efficiency.

Clarified viral vector particles can be concentrated using tangential flow filtration (TFF) and permeabilized into a suitable buffer, including the use of membranes with 1 to 100 nm pore size. This technology can greatly reduce the volume in a short time to concentrate virus particles, remove serum, degrade DNA fragments, *etc.* Most importantly, the operation with TFF is fully compliant with cGMP regulations. Since the liquid in TFF operation is parallel to the filter, the membrane clogging problem often encountered in other ultrafiltration methods can be greatly reduced, and the liquid flow rate can be kept at a high level. By TFF, approximately 90-100% of adeno-associated virus particles can be recovered. Currently, TFF can process approximately 100 L of viral products (Valkama, A.J. et al., (2020) Development of Large-Scale Downstream Processing for Lentiviral Vectors. Mol. Ther. Methods Clin. Dev. 17:717-730).

For pharmaceutical products used in human, viral vector products also need to be chromatographically purified. Anion exchange chromatography (AEX) is an efficient tool for viral vector purification, based on the positive charge characteristics of viral vectors at neutral pH. When the viral vector supernatant is passed through a column with a negatively charged matrix, the positively charged viral particles bind to the negatively charged matrix, and impurities flow directly through the column. Afterwards, the viral vectors are exposed to a high-salt environment (0.5-1M NaCl), and the bound particles are eluted from the anion-exchange column. High salt may inactivate virus particles, which is the only drawback of the technology. Exposure of virus particles to a high-salt eluent at room temperature inactivates 50% of the viruses (Segura, M.D.L.M. et al., (2005) A Novel Purification Strategy for Retrovirus Gene Therapy Vectors Using Heparin Afinity Chromatography. Biotechnol. Bioeng. 90:391-404).

Affinity chromatography is a purification technique used to separate biomolecules based on the specific interaction between target molecules and ligands attached to a chromatography column. Due to its high molecular selectivity, this technique can simplify the purification steps to a certain extent. Affinity chromatography can be divided into several categories according to the interaction, such as affinity chromatography based on hydrogen bonding, electrostatic interaction, van der Waals force, antibody-ligand interaction, *etc.* The antibody-ligand interaction is the most selective, and then affinity chromatography of antibodies binding to envelope proteins has not been used for viral vector purification. Heparin is a relatively cheap affinity ligand that interacts with a variety types of viruses and has been used in the purification of for example breast-associated virus vectors. In the purification, up to 53% of viral vectors can be recovered, and up to 94% of protein impurities and 56% of residual DNA can be removed. For the vectors and heparin, there is an interaction between positively charged molecules on the surface of virus particles and negatively charged heparin, so NaCl is usually used to elute the vectors from heparin column. The salt concentration typically needs to reach about 0.5 M to elute the viral vectors. This would require an additional cleaning or purification step to remove the salt, such as an additional TFF step.

In large-scale purification of viral vectors, size exclusion chromatography (SEC) can be used as a clean-up step to efficiently remove remaining impurities. SEC, also known as gel filtration chromatography, is used for viral vector purification based on the difference between the large volume of virus particles and the small volume of impurities. All impurities are smaller than the pore size and therefore can pass through the column and only the virus particles with large volume retain. The disadvantage of SEC is that it is difficult to scale up, and the loading volume is only 10% of the column volume. In addition, SEC purification requires a linear low flow rate and a long operating time.

In terms of AVV purification, cesium chloride density gradient centrifugation was first used. This method is time-consuming and labor-intensive, and has a low recovery rate, and cesium chloride is also potentially toxic to the human body. In 1998, Gimm *et al.* used specific antibodies against AAV structural proteins and developed an immunoaffinity chromatography purification method that can perform one-step purification of cell lysate and recover about 70% of AAV with a purity of about 80%. Since heparin is the natural receptor analog of AAV, AAV can be purified by heparin affinity chromatography. A potential problem with this approach is that many proteins also bind to heparin. One method is to first use iodixanol density gradient centrifugation and then perform heparin affinity chromatography, which can quickly and easily recover about 50-70% AAV with a purity of about 99%. Another method is lysing cells with deoxycholic acid, performing centrifugation to obtain a supernatant, preforming heating at 56°C for 45 min, performing centrifugation to remove precipitate after protein denaturation, and then performing heparin affinity chromatography. The method is also fast, reproducible, has a recovery rate higher than 70%, and a purity of up to 99%. Some laboratories have taken advantage of AAV's resistance to chloroform and proposed a three-step concentration and purification method of chloroform treatment-PEG/NaCl precipitation-chloroform extraction.

After the purification step, viral vectors may need to be frozen and stored at - 80°C.

The present application specifically relates to a method for preparing a viral vector particle by using the cell strain of the present application, including PowerS^{™}-293, and the method comprises the following steps:
i) culturing the above cell strain;
ii) introducing a viral vector expression system into the above cell strain;
iii) culturing the above cell strain under a condition favorable for the production of the viral vector particle; and
iv) collecting the viral vector particles.

Step i) may include suspension culture of the cell strain. In one embodiment, the cell strain is cultured with shaking in a serum-free medium under the condition of 37°C and 8% CO₂. The serum-free medium can be selected from LV-MAX production medium, Expi293 expression medium and OPM-293 CD03 medium. In one embodiment, the serum-free medium is OPM-293 CD03 medium.

Step i) may also include passaging of the cell strain.

Step ii) may include introducing the viral expression system into the cell strain via a physical or chemical transfection. The physical transfection can include electroporation, microinjection, gene guns, *etc.* The chemical transfection method can include calcium phosphate co-precipitation, polyethyleneimine (PEI)-mediated transfection, Lipofectamine (Invitrogen)-mediated transfection, *etc.*

The viral vector may be an adeno-associated virus vector. The adeno-associated virus vector can be AAV of all serotypes, and chimeras and hybrids thereof, for example, AAV1, AAV2, AAV3, AAV4, AAV5, AAV6, AAV7, AAV8, AAV9, AAV10, AAV11, AAV12, AAV13, and any chimeras and/or hybrids thereof, such as AAV1-13, *etc.* In one embodiment, the adeno-associated virus vector is, for example, AAV2, AAV5, AAV8, AAV9, AAV10, *etc.*

The viral vector expression system can include a heterologous nucleic acid-containing carrier plasmid, a rep-cap-containing packaging plasmid, and a helper plasmid. The helper plasmid can contain Ad5 genes, including VA, E2A and E4OEF6. The length of the heterologous nucleic acid in the carrier plasmid is no greater than 4.7 kb.

In one embodiment, the method is used to prepare an adeno-associated virus vector, including introducing three nucleic acid vectors into a cell strain, and the three nucleic acid vectors contain a rep-cap gene, an Ad5 gene (such as VA, E2A and E4OEF6), and a heterologous gene between two ITRs, respectively.

The heterologous nucleic acid can encode a protein of interest or an untranslated RNA. In one embodiment, the heterologous nucleic acid contains an open reading frame encoding a protein of interest or an untranslated RNA. The heterologous nucleic acid can encode a polypeptide or a protein to achieve overexpression of the polypeptide or the protein. The heterologous nucleic acid can encode shRNA and interfere with the expression of a target gene. The heterologous nucleic acid can encode gRNA and Cas9 to achieve knockout of a target gene. The heterologous nucleic acid can encode gRNA and dCas9 to achieve endogenous overexpression of a target gene. The carrier plasmid can contain a promoter that direct expression of a heterologous gene, such as a promoter that enable cell/tissue-specific expression.

Step iii) may include suspension culture of the cell strain. The cell strain can be cultured under any condition suitable for the cell strain to produce the viral vector, and the condition can be determined by those skilled in the art. In one embodiment, the cell strain is cultured with shaking in a serum-free medium under the condition of 37°C and 8% CO₂. The serum-free medium can be selected from LV-MAX production medium, Expi293 expression medium and OPM-293 CD03 medium.

Step iii) may also include passaging the cells.

The present application has carried out small-scale and pilot-scale production of PowerS^{™}-293 host cells containing a viral vector expression system, such as 300 ml shake flask culture, such as suspension culture in a 1 L, 2 L, 10 L and 50 L bioreactor (such as a WAVE reactor or a glass reactor) to produce the viral vector.

In step iv), the viral vector can be collected by lysing the cells, or the viral vector secreted by the cell strain can be collected from the medium.

The method can also include titer determination, purification, *etc.* of the viral vector after step iv).

### Application of viral vectors

A viral vector expression system may include a nucleic acid vector containing a heterologous nucleic acid encoding a therapeutic or research protein, or a therapeutic or research untranslated RNA such as siRNA. The application of viral vectors mainly depends on the protein and nucleic acid encoded by the heterologous nucleic acid.

The heterologous nucleic acid can encode a therapeutic molecule, such as a therapeutic peptide, or a therapeutic nucleic acid such as siRNA. A "therapeutic molecule" may be a peptide or protein that mitigates or alleviates symptoms resulting from protein deficiency or defect in a cell or subject. Alternatively, the "therapeutic" peptide or protein encoded by the heterologous nucleic acid is a substance that confers a benefit on a subject, such as correcting a genetic defect, correcting a gene (expression or function) defect, or providing an anticancer effect. The therapeutic nucleic acid can include, for example, siRNAs, antisense molecules, and miRNAs. The heterologous nucleic acid can encode a number of useful products.

The heterologous nucleic acid can encode hormones and growth and differentiation factors, including but not limited to insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor alpha (TGF-alpha), platelet-derived growth factor (PDGF), insulin-like growth factors I and II (IGF-I and IGF-II), any of the transforming growth factor beta superfamily, including TGFβ, kinetin, and inhibitors, or any of the bone morphogenetic proteins (BMPs) BMP1-15, nerve growth factor (NGF), brain-derived neurotrophic factor (BDNF), neurotrophins NT-3 and NT4/5, ciliary neurotrophic factor (CNTF), glial cell-derived neurotrophic factor (GDNF), aggregin, netrin-1 and netrin-2, hepatocyte growth factor (HGF), kainic acid, noggin, and tyrosine hydroxylase.

Other useful heterologous nucleic acid products include proteins that modulate the immune system, including but not limited to cytokines and lymphokines, for example thrombopoietin (TPO), interleukins (IL) IL-1 to IL-17, monocyte chemoattractant protein, leukemia inhibitory factor, granulocyte-macrophage colony-stimulating factor, Fas ligand, tumor necrosis factor alpha and beta, interferon alpha, beta and gamma, stem cell factor, flk-2/fFlt3 ligand, immunoglobulin IgG, IgM, IgA, IgD and IgE, chimeric immunoglobulin, humanized antibody, single-stranded antibody, T cell receptor, chimeric T cell receptor, single chain T cell receptor, G protein-coupled receptors (GPCRs) for example CCR5, Class I and Class II MHC Molecules, and engineered immunoglobulin and MHC molecules. Useful heterologous nucleic acid products also include regulatory proteins such as complement regulatory proteins, membrane cofactor proteins (MCPs), decay accelerating factors (DAFs), CR1, CF2, and CD59.

Other useful heterologous nucleic acid products include those gene products that can correct congenital metabolic defects, including but not limited to carbamyl synthetase I, ornithine transcarbamylase, arginosuccinate synthetase, arginosuccinate lyase, arginase, fumarylacetacetate hydrolase, phenylalanine hydroxylase, alpha-1-antitrypsin, glucose-6-phosphatase, porphobilinogen deaminase, clotting factor, for example Factor V, Factor VIIa, Factor VIII, Factor IX, Factor X, Factor XIII or protein C, cystathione beta-synthetase, branched chain ketoacid decarboxylase, albumin, isovaleryl-CoA dehydrogenase, propionyl-CoA carboxylase, methylmalonyl-CoA mutase, glutaryl-CoA dehydrogenase, Insulin, β-glucosidase, pyruvate carboxylate, hepatic phosphorylase, phosphorylase kinase, glycine decarboxylase, H protein, T protein, cystic fibrosis transmembrane regulator (CFTR) sequence and dystrophin cDNA sequence.

Other useful heterologous nucleic acid products include those that can make up for functional or activity defects, defective or absent, for example antibodies, retinal pigmented epithelial cell specific 65 kDa protein (RPE65), erythropoietin, low-density lipoprotein receptor, lipoprotein lipase, ornithine transcarbamylase, β-globulin, α-globulin, spectrin, α-anti-trypsin, adenosine deaminase (ADA), metal transporter (ATP7A or ATP7), sulfamidase, enzymes involved in lysosomal storage diseases (ARSA), hypoxanthine guanine phosphoribosyltransferase, beta-25 glucocerebrosidase, sphingomyelinase hexosaminidase, lysosomal hexosaminidase, branched chain ketoacid dehydrogenase, hormones, growth factors (for example, insulin-like growth factor 1 and 2, platelet-derived growth factor, epidermal growth factor, nerve growth factor, neurotrophins 3 and 4, brain-derived neurotrophic factor, glial cell-derived growth factor, transforming growth factor α and β, *etc*.), cytokines (for example alpha interferon, beta interferon, interferon gamma, interleukin 2, interleukin 4, interleukin 12, granulocyte macrophage colony-stimulating factor, lymphotoxin, *etc*.), products of suicide genes (for example, herpes simplex virus thymidine kinase, cytosine deaminase, diphtheria toxin, cytochrome P450, deoxycytidine kinase, tumor necrosis factors, *etc*.)*,* drug resistance proteins (for example, used in cancer therapy to confer drug resistance), tumor suppressor proteins (for example p53, Rb, Wt-1, NF1, VonHippel-Lindau (VHL), adenomatous polyposis (APC)), immunomodulatory peptide, tolerance or immunogenic peptides or proteins Tregitopes, or hCDR1, insulin, glucokinase, guanylate cyclase (LCA-GUCY2D), Rab guard protein 1, LCA5, ornithine ketoacid aminotransferase, Retinoschisis 1 (X-linked retinoschisis), USH1C (Usher syndrome 1C), X-linked retinitis pigmentosa GTPase (XLRP), MERTK (AR form of RP: retinitis pigmentosa), DFNB1 (Connexin26 deafness), ACHM2, 3 and 4 (colorblind), PKD-1 or PKD-2 (polycystic kidney disease), TPP1, CLN2, genetic defects caused by lysosomal storage diseases (for example, sulfatase, N-acetylglucosamine-1-phosphotransferase, Cathepsin A, GM2-AP, NPC1, VPC2, sphingolipid activator proteins, *etc*.)*,* one or more zinc finger nucleases for genome editing, or donor sequences used as repair templates for genome editing.

Alternatively, the heterologous nucleic acid can include, for example, siRNAs, antisense molecules, and miRNAs. Clinically useful adeno-associated virus vectors can also express antisense genes against human immunodeficiency virus (HIV) (Levine BL et al., (2006) Gene transfer in humans using a conditionally replicating lentiviral vector. Proc Natl Acad Sci U S A. 103(46): 17372-7) and other important human pathogens. Naldini (Naldini L. (2011) Ex vivo gene transfer and correction for cell-based therapies. Nat. Rev. Genet. 12:301-315) discusses and cites these and other useful applications of adeno-associated viruses and related vectors. The antisense gene contained in the adeno-associated virus vector inhibit the expression of: Huntington's (HTT) gene, a gene associated with dentatorubral-pallidoluysian atrophy (for example atrophin 1, ATN1); X-chromosomal androgen receptor in spinobulbar muscular atrophy, human Ataxin-1, -2, -3 and -7, (CACNA1A) encoded Cav2.1P/Q voltage-dependent calcium channel, TATA-binding protein, Ataxin8 opposite chain also known as ATXN8OS, isoform of serine/threonine protein phosphatase 2A55kDa regulatory subunit Bβ in spinocerebellar ataxia (type 1, 2, 3, 6, 7, 8, 12, 17), FMR1 in fragile X syndrome (fragile X mental retardation 1), FMR1 in fragile X-related tremor/ataxia syndrome (fragile X mental retardation 1), FMR1 (fragile X mental retardation 2) or AF4/FMR2 family member 2 in fragile X mental retardation; myotonic protein kinase (MT-PK) in myotonic dystrophy; frataxin in Friedrich's ataxia; a mutations in the superoxide dismutase 1 (SOD1) gene in amyotrophic lateral sclerosis; a gene involved in the pathogenesis of Parkinson's and/or Alzheimer's disease; Apolipoprotein B (APOB) and subtilisin precursor protein convertase/kexin type 9 (PCSK9), hypercholesterolemia; HIVTat and human immunodeficiency virus trans-activating factor of transcribed genes in HIV infection; HIVTAR, HIVTAR and human immunodeficiency virus trans-activating factor response element genes in HIV infection; C-C chemokine receptor (CCR5) in HIV infection; Rous sarcoma virus (RSV) in RSV infection, liver-specific microRNA (miR-122) in hepatitis C virus infection; p53, acute kidney injury, or renal transplantation with delayed graft function, or renal injury with acute renal failure; protein kinase N3 (PKN3) in progressive or metastatic solid tumors; LMP2; LMP2 is also known as proteasome subunit beta type 9 (PSMB9), metastatic melanoma; LMP7, also known as proteasome subunit beta type 8 (PSMB8), metastatic melanoma; MECL1, also known as proteasome subunit beta type 10 (PSMB10), metastatic melanoma; vascular endothelial growth factor (VEGF) in solid tumors; spindle kinesin in solid tumors, apoptosis suppressing B-cell CLL/lymphoma in chronic myeloid leukemia (BCL-2), nucleotide reductase M2 (RRM2) in solid tumors, Furin in solid tumors; Polo-like kinase 1 (PLK1) in liver tumors, diacylglycerol acyltransferase 1 (DGAT1) in hepatitis C virus infection, β-catenin in familial adenomatous polyposis; beta 2-adrenergic receptors, glaucoma; RTP801/Redd1, also known as DNA damage-induced transcription factor 4 protein, in diabetic macular edema (DME) or age-related macular degeneration; vascular endothelial growth factor receptor I (VEGFR1) in age-related macular degeneration or choroidal neovascularization, caspase 2 in non-arteritic ischemic optic neuropathy; keratin 6AN17K mutant protein in pachyonychia congenita; influenza A virus A genome/gene sequence in influenza infection; severe acute respiratory syndrome (SARS) coronavirus genome/gene sequence in SARS infection; respiratory syncytial virus infection genome/gene sequence in respiratory syncytial virus infection; Ebola filovirus genome/gene sequence in Ebola virus infection; hepatitis B and C virus genome/gene sequence in hepatitis B and C virus infection; herpes simplex virus (HSV) genome/gene sequence in HSV infection, Coxsackievirus B3 genome/gene sequence in Coxsackievirus B3 virus infection; torsin-like A (TOR1A) silencing pathogenic allele (allele-specific silencing) in primary dystonia, pan-class I and HLA allele in transplantation; mutated rhodopsin gene (RHO) in autosomal dominant hereditary retinitis pigmentosa (adRP); or inhibitory nucleic acids that bind to transcripts of any of the aforementioned genes or sequences.

In addition, studies have shown that the heterologous nucleic acid can encode shRNA and interfere with the expression of a target gene. The heterologous nucleic acid can encode gRNA and Cas9 to achieve knockout of a target gene. The heterologous nucleic acid can encode gRNA and dCas9 to achieve endogenous overexpression of a target gene.

### Screening of host cell lines

Selecting a suitable host cell can greatly increase the production of viral vectors. Preferred cells are those suitable for serum-free culture, and/or adapted to suspension culture. As mentioned above, if a serum-free medium is used for cell culture and proliferation, potential immunogenic substance contamination and animal-derived components in the final product can be reduced, and the downstream purification process can be simplified, and the simplification of the purification process can improve the yield and activity of viral vectors. In addition, suspension culture is more suitable for large-scale production of viral vectors than adherent culture.

### Serum-free culture

Generally speaking, the growth of animal cells depends on the presence of serum. Most cells cannot proliferate without the addition of serum. Serum medium is usually supplemented with bovine serum.

The composition of serum is complex, including proteins that assist in the transportation of substances, nutrients, hormones and factors that promote cell growth, *etc.* Because of the complexity of the natural components of serum, serum plays a variety of functions in the process of cell culture, including providing hormones to maintain cell growth and promoting the growth of cultured cells; supplementing nutrients that are absent or in a small amount in the basal medium; providing binding proteins to promote cell recognition and utilization of vitamins, lipids and other hormones; in some cases, the binding proteins can bind to toxic metals and pyrogens to detoxify; promoting cell adherence and providing factors that can promote cell spreading on a plastic culture matrix; acting as a pH buffer; and providing protease inhibitors to inactivate residual trypsin during cell digestion and protect cells from damage. However, the use of bovine serum has the risk of contaminating exogenous viruses and becoming a pathogenic factor, and due to the inconsistency of biological activities and factors between different batches of bovine serum, the reproducibility of products and experimental results is poor, and the residual bovine serum in products is also easy to cause allergic reactions in viral vector recipients to the serum.

Serum-free medium, referred to as SFM, is a cell medium without adding serum. It is the third type of medium after natural medium and synthetic medium. Compared with a traditional medium, the serum-free medium does not contain animal serum or other biological extracts, but it can still maintain the growth and reproduction of cells *in vitro* for a long time. Due to its relatively clear composition and simple preparation process, SFM has been widely used in the field of modern biotechnology, and it is also a powerful tool for elucidating basic research issues of cell growth, proliferation, differentiation, and gene expression regulation.

There are currently two types of serum-free media, one does not contain any added components of animal origin, and the other one does not contain any unspecified added components. At present, the following four serum-free media are widely used.

The first one is the serum-free medium in a general sense, which uses various biological materials (such as bovine serum albumin, transferrin, insulin and other biological macromolecules, and protein-free mixed lipids extracted from serum and hydrolyzed proteins) that can replace the function of serum to prepare cell medium. Its characteristic is that the protein content in the medium is relatively high, but the chemical composition of the added substances is not clear, and it contains a large amount of animal-derived proteins.

The second one is an animal source-free medium. The animal source-free medium developed by many commercial companies is based on safety considerations for the production of recombinant drugs. The added components in the medium do not have components of animal origin, and the required proteins are derived from recombinant proteins or protein hydrolyzates. Such components can ensure the needs of cell growth and proliferation.

The third one is an animal protein-free medium. The medium does not use animal-derived proteins at all, but there are still some added components derived from hydrolyzed fragments of plant proteins or synthetic polypeptide fragments and other derivatives. Such a medium has a relatively stable composition, but must be supplemented with steroid hormones and lipid precursors, and is highly specific to the cells being cultured.

The fourth one is a chemically defined medium. Such a medium is currently the safest and most ideal medium, and can ensure the consistency between batches of medium, and the added small amount of animal-derived protein hydrolyzates and proteins are all defined components. Its characteristic is that the nature of the medium is determined, and it is convenient to formulate the medium.

The advantages of the serum-free medium include avoiding quality variation between serum batches and improving the reproducibility of cell culture and experimental results; avoiding the toxic effect of the serum on cells and serum-derived contamination; avoiding the influence of serum components on experimental research; conducive to the differentiation of cultured cells *in vitro*; improving the expression level of the product and making the cell product easy to purify; having a stable composition and production in large quantities; containing no mitogen inhibitors, and promoting cell proliferation, *etc.* The disadvantage of such a medium is that cells are susceptible to certain mechanical and chemical factors in the serum-free medium, and the preservation and application of the medium are not as convenient as the traditional synthetic medium; high cost; very specific, that is, one serum-free medium is only suitable for the culture of a certain type of cells; cells at different differentiation stages of development require different formulations, and the selection of growth factors and cytokines is particularly important. Moreover, while removing the serum, the protective effect provided by some serum proteins is also removed, so the requirements for the purity of reagents and water and instrument cleanliness are high.

The serum-free medium is made from a nutrient-complete basal medium supplemented with hormones, growth factors, adherence factors, and binding proteins.

The basal medium used in cell culture in the early days includes natural medium such as plasma clot, lymph fluid, soybean peptone and embryo extract. In 1950, Morgan *et al.* developed 199 medium on the basis of previous researches, which marked a new stage of animal cell medium and the stage of synthetic medium. The synthetic medium is a basal medium composed of a certain proportion of amino acids, vitamins, inorganic salts, glucose, *etc.* according to the needs of cell growth. At present, there are hundreds of synthetic media commercially available on the market. Among many synthetic media, MEM, DMEM, RPMI 1640, F12, and TC100 are the most widely used. The composition of the basal medium is well known, so when culturing different cell strains, some components of the basal medium can be adjusted accordingly to better meet the nutritional requirements of the cell strains or increase the expression of proteins of interest.

Added factors in the serum-free medium, also known as supplementary factors, are the general term that replace various serum factors. Most serum-free culture media must be supplemented with 3 to 8 factors, and any single factor cannot replace serum. More than 100 such factors are known, some of which are essential supplementary factors, such as insulin, sodium selenite, and transferrin, and most of the others are accessory factors. According to different functions, supplementary factors can be divided into four categories.

The first category of the supplementary factors are hormones and growth factors. Hormones, such as insulin, growth hormone and glucagon, need to be added when many cells are cultured in the serum-free medium. Almost all cell lines require insulin, which is a polypeptide that can bind to an insulin receptor on a cell to form a complex, promote the synthesis of RNA, proteins and fatty acids, and is an important cell survival factor. In the serum-free culture of cells, the concentration of insulin used is 0.1-10 µg/ml. Jan *et al.* believed that the rapid depletion of insulin in batch culture is the main reason for the decrease of cell specific growth rate. In addition, thyroxine and steroid hormones such as progesterone, hydrocortisone, and estradiol are also commonly used supplementary factors for serum-free cell culture. Different cell strains require different types and quantities of hormones. Growth factors are essential supplementary factors to maintain the survival, proliferation and differentiation of cultured cells *in vitro.* According to their chemical properties, they can be divided into polypeptide growth factors and steroid growth factors. The growth factors added to the serum-free medium are mainly polypeptide growth factors. In recent years, 20-30 kinds of polypeptide growth factors have been identified, and for more than half of them, corresponding recombinant growth factors can be obtained through genetic recombination. The common growth factors in the serum-free medium include epidermal growth factor (EGF), fibroblast growth factor (FGF) and nerve growth factor (NGF). Growth factors are potent mitogens that shorten cell population doubling time.

The second category of the supplementary factors are binding proteins. There are two binding proteins, one is transferrin and the other one is albumin. There are specific transferrin receptors on most mammalian cells. The binding of the receptors and the complex of transferrin and iron ions is the main source for cells to obtain the essential trace element iron. In addition, transferrin also has the properties of growth factors and can bind to other trace elements such as vanadium. Different cells need different amounts of transferrin. Albumin is also a commonly used added factor in the serum-free media. It can bind with vitamins, lipids, hormones, metal ions and growth factors to stabilize and regulate the activity of the above substances in the serum-free medium, in addition it can bind to toxins and reduce the influence of proteases on cells.

The third category of the supplementary factors are adherence factors. Most eukaryotic cells require adherence to a suitable substrate for growth *in vitro.* Cell adherence is a complex process involving the adsorption of adherence factors on the surface of a vessel or carrier, the binding of cells with the adherence factors, *etc.* Adherence factors commonly used in serum-free culture include intercellular substances and serum components, such as fibronectin, collagen, laminin, polylysine, *etc.* In the suspension culture as described in the present application, for example, adherence factors do not need to be added to the medium.

For serum-free culture of some cell lines, it is necessary to supplement some low molecular weight chemicals, such as trace elements, vitamins, lipids, *etc.* Vitamin B mainly participates in cell metabolism in the form of coenzyme, and vitamin C and vitamin E have antioxidant effects. Butanediamine and linoleic acid provide lipids needed for cell membrane synthesis and water-soluble lipids needed for cell growth.

At present, the serum-free medium has been widely used in large-scale animal cell culture. In the application field of biological products such as vaccine growth, monoclonal antibodies and various biologically active proteins, optimizing the composition of the serum-free medium can enable different cells to maintain high-density culture in an environment that is most conducive to cell growth and expression of products of interest, thereby reducing manufacturing cost. During human cell culture, the use of the serum-free media can also selectively control and avoid the overgrowth of fibroblasts. Under serum-free culture conditions, the growth of certain cells and the production of antibodies are even several times higher than those with serum.

In addition to the production of biological products, the serum-free medium is also widely used in the fields of cell biology, pharmacology, and oncology. For example, the serum-free medium can be used to study the differentiation conditions of cells. The composition of the serum-free medium can be well known chemical substances, so the types and quantities of important biologically active substances can be increased or decreased according to the needs of research. This provides an effective means for studying the conditions of cell differentiation. Another example is that the serum-free medium can be used to select target cells from mixed culture of a variety of cells. By selecting certain components in serum-free culture, the excessive growth of non-target cells in primary tissue culture can be inhibited, and the purpose of selecting target cells can be achieved. Serum-free culture is also used in the study of tumor pathology and etiology. For example, it is used to study the influence of carcinogens on cells, to study the ability of tumor cells to respond to peripheral signals that may trigger the terminal differentiation of normal cells, to study the relationship between the growth and migration of normal cells and tumor cells and basement membrane signals, *etc.* In addition, the serum-free culture can also be used to study the interaction of hormones, growth factors and drugs with cells.

Commercially available serum-free cell growth media include FreeStyle^{™} 293 (Gibco^{™}, Life Technologies), DMEM/F12 (Gibco^{™}, Life Technologies), SFM4Transfx-293 (HyClone^{™}, ThermoScientific), CDM4HEK293 (HyClone^{™}, ThermoScientific), StemPro-34SFM (Gibco^{™}, Life Technologies), FreeStyle F17 (Gibco^{™}, Life Technologies), 293SFMII (Gibco^{™}, LifeTechnologies), CD293 (Gibco^{™}, LifeTechnologies), LV-MAX (A35834-01, Gibco^{™}), Expi293 (Gibco^{™}, A14351-01), EX-CELL^{®}293 (Sigma, 14571C-1000 mL), BanlanCD HEK293 (Irvine, 91165), Pro293s-CDM (Lonza, 12-765Q), OPM-293 CD03 (Shanghai OPM Biosciences Co., Ltd., 81070-001), and Celkey CD HEK293 (JSBiosciences, 10804-19008) medium. Herein, these serum-free media can be used for screening and/or culturing cells.

### Suspension culture

Suspension culture refers to dispersing and suspending cells in the medium for the culture of single cells and small cell clumps and is a culture method of adherence-independent cells. Certain adherence-dependent cells can also be cultured by this method after adaptation and selection. Scaling up the suspension culture is relatively simple, simply by increasing the volume. When the depth of the medium exceeds a certain value, it is necessary to shake the medium by a shaking or rotating device. When the depth of the medium is deeper, carbon dioxide and oxygen need to be introduced to ensure sufficient gas exchange.

Suspension culture process is divided into suspension cell culture process and adherent cell microcarrier suspension culture process according to cell adherence. Suspension cells (CHO cells, BHK21l cells, HEK293 cells, *etc.)* can be directly produced and proliferated in a reactor, the cells grow freely, the culture environment is uniform, the sampling is simple, the culture operation is simple and controllable, the scaling-up is convenient, the contamination rate and the cost are low; however, the suspension culture of adherent cells in the reactor requires the use of microcarriers, and the nutrient environment at the contact site between the cells and the spheres is poor, and the culture, sampling for observation and scaling-up processes are complex and costly.

The cell suspension culture process is divided into batch culture, fed-batch culture and perfusion culture according to the culture method. The batch culture can intuitively reflect the growth and metabolic changes of cells in a bioreactor, and the operation is simple, but there are many metabolic waste products in the initial stage, which inhibits cell growth and the cell growth density is not high; the fed-batch culture is simple to operate, has high yield, is easy to scale up, and is widely used, but it is necessary to design a fed-batch medium; The culture volume of the perfusion culture is small, the recovery volume is large, the residence time of the product in a tank is short, and the product can be recovered in time and stored at a low temperature, which is beneficial to maintain the activity of the product; but the operation is complicated, the utilization efficiency of the cell medium is low, and the rotary filter is easy to block, *etc.* For different viral vectors, different cell culture methods are used. For example, for a biological product that is secreted and whose activity decreases rapidly, the perfusion culture should be used, and the perfusion culture is also more suitable for microcarrier culture.

After selecting a suitable production process, process control becomes the key. In order to ensure that the cells grow in an optimal environment, it is necessary to use the online monitoring function of the reactor to control various operating parameters. The cells are sensitive to temperature, and appropriate heating or cooling methods are required to control the culture temperature at 35°C to 37°C to avoid cell damage. The suitable pH range for animal cell growth is generally between 6.8 and 7.3, and any pH lower than 6.8 or higher than 7.3 may have adverse effects on cell growth.

Whether the scale of cell culture can be enlarged is an important prerequisite for selecting a reactor. The scaling up of the suspension culture is mainly through increasing the volume of the reactor or increasing the number of reactors. Increasing the volume of the reactor can save a lot of supporting engineering and personnel costs, while multiple small reactors, although flexible in operation, are relatively expensive. In the production of biological products abroad, large-scale bioreactors that can achieve scaling-up step by step are widely used. The selection and configuration of bioreactors also need to consider and meet the production process and capacity requirements. The standardization of the reactor interface, the speed of supply of accessories, and the quality of after-sales service should all be considered factors in the selection of bioreactors for industrialization, so as to avoid delays in production.

In the process of liquid suspension culture, attention should be paid to the subculture of cells in time, because when the cells grow to a certain period, they would enter the stationary phase of division. For most suspension cultures, the cells reach their maximum density on days 18 to 25, at which time the first subculture should be performed. When subculturing, large cell clumps and inoculum debris should be removed.

### Screening of cell strains

The essence of cell screening and adaptation is to apply modern cell biology techniques to perform study on adapting to different culture modes (such as suspension) and different media (such as serum-free media), reducing cell apoptosis rate, improving cell vitality, prolonging cell life cycle and increasing product concentration, so as to screen a cell strain suitable for production. In order to achieve a stable adaptation, the high density cell culture is usually shifted to low density cell culture, and the high concentration serum culture is gradually shifted to low serum concentration culture. Because cells are difficult to repair after damage, the cell viability should be kept more than 90% during adaptation. The proliferative ability of cells directly affects the production capacity, and the determination of their proliferative ability should be maintained during adaptation. When cells are adapted, their expression and secretion ability should also be determined to avoid changes in the expression characteristics of the cells after adaptation. The proliferative ability, vitality and production ability of the adapted cells should be able to meet the needs of large-scale industrial production. Due to the different nutritional requirements of specific cells, the degree of difficulty in achieving adaptation is also different. During adaptation, it is necessary to select a suitable cell medium and supplement certain nutrients in a targeted manner to meet the specific needs of cells so that the adapted cells can keep their characteristics of suspension or serum-free growth.

Also, in suspension culture, cells tend to clump. Cell clumping prevent many of the plasmids used for vector production from transfecting cells, and cells in the middle of large clumps die from lack of nutrients. At present, the method of improving cell clumping is mainly by adding an anti-clumping agent. The addition of the anti-clumping agent may significantly reduce the transfection efficiency, resulting in cells that cannot be transfected and cannot package adeno-associated viruses.

There is an urgent need in the field for an efficient and convenient method for adapting/screening a cell strain with high dispersibility, high proliferation density, and high production of viral vectors.

In the present application, the inventors mainly improved the adaptation/screening method for HEK293 cells, which are currently the most used in adeno-associated virus preparation.

First of all, the inventors do not adopt the culture step of gradually reducing the serum concentration from high concentration serum culture commonly used in the field, but directly add the adherent cells cultured in an ordinary medium to a serum-free medium for suspension culture, which greatly saves time and may also be helpful for successfully screening out a cell strain with high dispersibility (that is, not easy to clump), high proliferation density and high viral vector production.

In addition, the inventors of the present application also found that using several specific serum-free suspension media, such as LV-MAX production medium (Gibco, A35834-01), Expi293 expression medium (Gibco, A14351-01) and OPM-293 CD03 medium (Shanghai OPM Biosciences Co., Ltd., 81070-001), is more conducive to the screening of a cell strain with high dispersibility and without clumping. Specifically, the cells screened from these three media can reach a high density (~1 × 10⁷ cells/ml) and the proportion of clumped cells is relatively low, which is conducive to the screening of subclones without clumping. Among these three media, OPM-293 CD03 medium (Shanghai OPM Biosciences Co., Ltd., 81070-001) has the best screening effect.

The present application screens mainly HEK293 cells, which are commonly used in the preparation of adeno-associated virus vectors, have fast proliferation, high transfection efficiency, and efficient production of viral vectors, and have the potential for large-scale production of viral vectors.

Specifically, the present application provides a method for screening or adapting HEK293 cell strains adapted to serum-free suspension culture, comprising:
i) taking a HEK293 cell adherently cultured in a serum-containing medium, and placing the cell in a serum-free medium for suspension culture; and
ii) selecting a monoclonal cell strain adapted to serum-free suspension culture.

The serum-containing medium in step i) may contain 10% serum, such as 10% fetal bovine serum.

The suspension culture in step i) may include shaking culture at a condition of 37°C and 8% CO₂.

The suspension culture in step i) may include cell passaging.

The serum-free medium can be selected from LV-MAX production medium (Gibco, A35834-01), Expi293 expression medium (Gibco, A14351-01) and OPM-293 CD03 medium (Shanghai OPM Biosciences Co., Ltd., 81070-001), preferably OPM-293 CD03 medium (Shanghai OPM Biosciences Co., Ltd., 81070-001).

Step ii) may include selecting a monoclonal cell strain with high dispersibility (that is, non-clumping) and adapted to serum-free suspension culture.

Step ii) may include selecting monoclonal cells through at least one round, for example two rounds, of limiting dilution and photographing and imaging, and culturing the selected monoclonal cells in suspension.

Culturing the selected cell in suspension in step ii) may include shaking culture at a condition of 37°C and 8% CO₂. Culturing the selected cell in suspension in step ii) may include cell passaging.

During the implementation of this method, no anti-clumping agent needs to be added.

In the method of the present application, 1) the cells adherently cultured in the serum-containing medium are directly subjected to serum-free suspension culture, and there is no step of progressive serum decreasing, which can greatly shorten the adaption and screening process, and the adaption and screening period can be shortened to about 20 days; 2) the serum-free medium can be used to culture the cells, avoid potential immunogenic substance contamination and animal-derived components, and simplify the downstream purification process; 3) the serum-free medium selected from LV-MAX production medium, Expi293 expression medium and OPM-293 CD03 medium, especially OPM-293 CD03 medium, is used, and is more conducive to screening the HEK293 cell strain suitable for suspension culture, that is, with high proliferation density and high dispersibility; 4) the use of the anti-clumping agent is avoided, and the transfection efficiency of the cells is not affected; and 5) two rounds of limiting dilution and imaging ensure the monoclonal origin, ensure that the cells provide stable virus expression quantity, and also provide intuitive data support for the monoclonal origin.

### limiting dilution and photographing and imaging

After suspension adaption, the adaptability, growth rate, and virus-producing ability of each HEK293 cell to this culture method are not uniform, which results in low overall levels of cell growth and virus production. Monoclonal origin is a necessary condition for product quality and expression quantity stability, and it is necessary to screen out clones that do not clump and have strong virus-producing ability. If the selected monoclonal is not a "real" monoclonal, the expression quantity of virus in different cell generations may be unstable when the clone is used in the later virus production test experiment, which would seriously affect the quality and stability of the later products.

The current methods for screening monoclonal cells include (1) two rounds of limiting dilution to control the diluted cell density to be less than 0.5 cells/well; (2) one round of limiting dilution plus monoclonal imaging and photographing; (3) FACS plus one round of limiting dilution or monoclonal imaging and photographing; and (4) semi-solid culture. The existing problem is that the cell viability after FACS sorting is low, and it is not feasible to photograph HEK293 cells selected in semi-solid medium, and data on the monoclonal origin cannot be provided.

Currently, a method of one round of limiting dilution plus monoclonal imaging is used to screen monoclones.

The limiting dilution method is a cell cloning method routinely used in laboratories. However, the limiting dilution method may add multiple cells in one well, so multiple subcloning is required to increase the probability of monoclonality.

Imaging technology provides a very intuitive way to provide supporting data to ensure the clonal origin of producer cell lines and can replace some additional laboratory work. However, there are factors that affect the validity (and "accuracy") of an image, such as calibration, focus, lighting, depth of focus, resolution, *etc.*

In the present application, at least one round, for example two rounds, of limiting dilution plus monoclonal imaging can not only provide intuitive data support for the monoclonal origin, but also make up for the shortcomings of imaging technology to a certain extent, greatly increasing the probability of monoclonals.

### Cell strain for preparing AVV of the present application

Starting from the HEK293 cells purchased from ECACC, a variety of cell strains with high proliferation density and high dispersibility were obtained through the cell strain screening/adaptation method of the present application.

One of them, named PowerS^{™}-293, was deposited at the China General Microbiological Culture Collection Center (CGMCC) on August 9, 2021 according to the Budapest Treaty with a deposit number of CGMCC No.: 23020.

The PowerS^{™}-293 cell strain can grow in suspension culture in a serum-free medium. The serum-free medium can be selected from LV-MAX production medium, Expi293 expression medium and OPM-293 CD03 medium, especially OPM-293 CD03 medium.

Cell clumping can not appear during the suspension culture of the PowerS^{™}-293 cell strain.

PowerS^{™}-293 cell strain can be used to produce viral vectors, such as adeno-associated viruses.

PowerS^{™}-293 cell strain can grow in suspension culture in the serum-free medium, with high proliferation density, good viability, stable virus production capacity after passage, and no cell clumping, that is, with high dispersibility. Therefore, no additional anti-clumping agent is needed in the suspension culture process.

The screened/adapted PowerS^{™}-293 cell strain was tested for the following indicators, and all met the indicators:
1) Cell viability > 90%;
2) Doubling time of 25-30 h;
3) The cell density in the logarithmic phase of 3-4 × 10⁶ cells/ml;
4) Maximum density > 1 × 10⁷ cells/ml;
5) The proportion of clumped cells of < 10%, and the number of cells in each cell clump of ≤ 5;
6) The titer of packaged adeno-associated virus of ≥ 1 × 10¹¹ Vg/ml;
7) The PCB (original cell bank) has completed comprehensive testing for sterility, mycoplasma, *etc*.;
8) Cell stability ≥ 50 PD1 (population doubling level).

Specifically, the doubling time of PowerS^{™}-293 cells in OPM-293 CD03 medium is short, about 24 h, and the cell density at logarithmic phase can reach 1.31 × 10⁷ cells/ml, and the highest density is more than 1.6 × 10⁷ cells/ml, and the virus-producing ability of the cell would not decrease with the increase of passage times (at least within 20 generations).

Compared to commercially available virus producer cells, PowerS^{™}-293 produces more viral vector particles after transfection with viral plasmids.

The technical solutions of the present invention are further illustrated in more detail by the examples and in conjunction with the accompanying drawings. Unless otherwise stated, the methods and materials in the examples described below are conventional products that can be purchased from the market. A person skilled in the art of the present invention would understand that the methods and materials described below are exemplary only and should not be considered as limiting the scope of the present invention.

### Example 1. Serum-free suspension adaption of HEK293 cells

The HEK293 cells purchased from ECACC were incubated in a 37°C water bath for about 1 min, and the cells in the cryovial were quickly thawed until there was no ice in the cryovial.

The cells were transferred from the cryovial to a sterile centrifuge tube, 4-5 milliliters of pre-warmed DMEM (containing 10% FBS) were added, and the mixture was centrifuged at 200 g for 5 min.

The supernatant in the culture flask was discarded and the cell pellet was resuspended in 3 ml of DMEM (containing 10% FBS). Based on the cell count results, the required number of cells were added to the T75 culture flask, and the T75 culture flask was placed in an incubator at 37°C and 5% CO₂.

The cells were incubated for three days and the cell medium in the flask was discarded when the cells were > 90% confluent. The degree of cell confluency herein refers to the percentage of the surface area of the culture vessel covered by the monolayer of the cells observed under a microscope for the cells grown in a monolayer. The adherent layer was rinsed once with DPBS. The cells were incubated with 0.25% trypsin (Company: Gibco, Cat. No.: 12563-029) for 2 min, after the cells were detached, 4-5 ml of DMEM containing 10% FBS was added to the T75 culture flask. The cells were transferred to a sterile centrifuge tube and centrifuge at 200 g for 5 min.

The supernatant was aspirated and the cell precipitate was resuspended in 6 ml of DMEM (containing 10% FBS). Based on the cell count results, the required number of cells were added to the T75 culture flask, and the T75 culture flask was placed in an incubator at 37°C and 5% CO₂.

The cells were passaged when they were fully adapted to adherent growth and reached > 90% confluency.

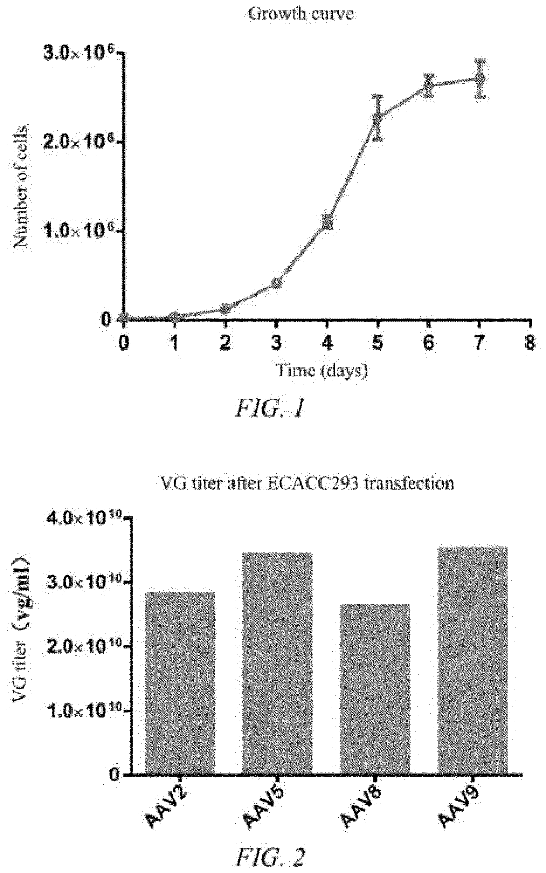

The growth curve test was performed after the cells were passed to 3 generations. As shown in Fig. 1, in the 7-day growth curve test, the adherent cells had a doubling time of 17 h (less than 24 h), and had good growth status and can be used for suspension adaption.

At the same time, AAV2, AAV5, AAV8, and AAV9 virus packaging tests were performed on adherent cells to observe the packaging ability of adherent HEK293 cells. When the cells grown evenly and the confluency was ≥ 90%, transfection can be performed. Otherwise, the culture should continue for no more than 24 h, and the culture time was recorded. Replacement to 9 mL of DMEM was performed before transfection.

Firstly, the transfection complex solution A was prepared. Specifically, according to the molar ratio of carrier plasmid (pAAV-EGFP): packaging plasmid (pRC2/pRC5/pRC8/pRC9): helper plasmid (pHelper) = 1 : 1 : 1, each plasmid was added into a 15 mL centrifuge tube filled with 0.5 ml of transfection medium DMEM according to the quantity in Table 1, gently pipetted for 3-5 times to make the plasmids mixed evenly. The centrifuge tube was marked as A, and all the vector sequences were from addgene and synthesized by GenScript.

Then transfection complex solution B was prepared. Specifically, PEI pro was added into a 15 mL centrifuge tube filled with 0.5 ml of transfection medium DMEM in the volume ratio of transfection reagent: DNA = 2 : 1, that is, at 30 µl, as shown in Table 1, and gently pipetted for 3-5 times to make them evenly mixed, and the centrifuge tube was marked as B.

**Table 1. Transfection complex composition**

| Total DNA amount | Solution A | | | |
|---|---|---|---|---|
| | pAAV-EGFP | pRC2 | pHelper- | DMEM |
| | | pRC5 | | |
| | | pRC8 | | |
| | | pRC9 | | |
| 15 µg | 3.6 µL | 4.4 µL | 7 µL | 0.5 mL |

| Transfection reagent: DNA | Solution B | | | |
|---|---|---|---|---|
| 2 : 1 | PEIpro | | | DMEM |
| | 30 µL | | | 0.5 mL |

Solution B was added to solution A, and the mixture was gently pipetted 8-10 times to make them evenly mixed, and let stand for 15 min to prepare a transfection complex with a total volume of 1.0 mL.

After standing for 15 min, 4 transfection complex mixtures were slowly and evenly added to 4 T75 culture bottles, with 1.0 ml in each T75 culture bottle. After 6-8 h, liquid replacement was performed, that is, the old medium was aspirated from the flasks and discarded, and replaced with 15.0 mL of DMEM containing 2% FBS. After 72 h, the virus was harvested for titer testing.

As shown in Fig. 2, the average packaging titer of adherent cells is 2.5 × 10¹⁰ vg/ml (the unit means the number of copies of the target genome GFP contained in each ml of virus liquid), which can be compared to packaging results of monoclonal cells after suspension adaption.

The cells were then transferred to serum-free medium (SFM), including LV-MAX production medium (Company: Gibco, Cat. No.: A35834-01), Expi293 expression medium (Company: Gibco, Cat. No.: A14351-01), FreeStyle^{™}293 expression medium (Company: Gibco, Cat. No.: 12338-018), BanlanCD HEK293 medium (Company: Irvine, Cat. No.: 91165), OPM-293 CD03 medium (Company: Shanghai OPM Biosciences Co., Ltd., Cat. No.: 81070-001), and serum-free culture was performed. Specifically, the cells with a confluency of more than 85% in the T75 culture flask were digested with 0.25% trypsin, and then centrifuged at 200 g for 5 min. The supernatant was aspirated and fresh SFM was added to resuspend the cells, the cells was transferred to a 125 ml shake flask which was placed in a 37°C, 8% COx incubator for culture on a shaker with a shaking diameter of 19 mm and a rotation speed of 125 rpm. When the cell density reached 3-6 × 10⁶ cells/ml, the cells were passaged, and the inoculated cells had a density of 0.35-0.55 × 10⁶ cells/ml, and cultured for 3-4 days until the survival rate exceeded 80%.

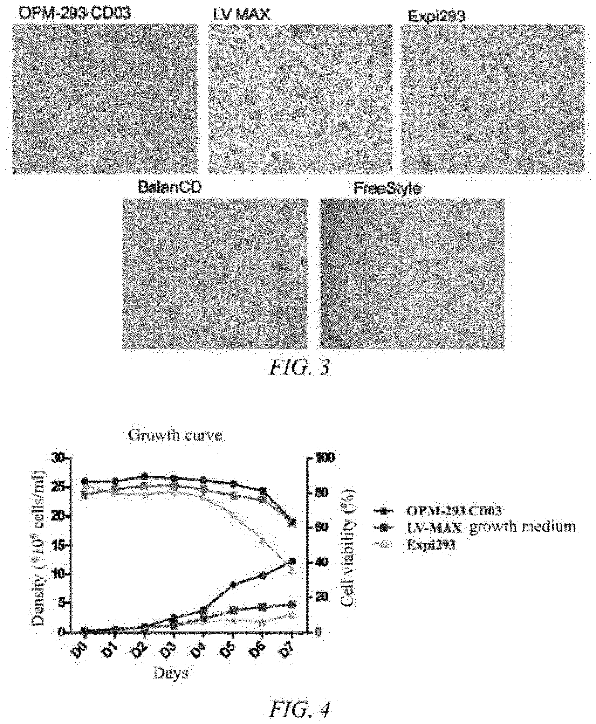

As shown in Fig. 3, adherent ECACC293 cells could proliferate normally after being suspended and adapted in OPM-293 CD03, LV-MAX and Expi293 medium for 3 generations, and the cell viability exceeded 80%. However, the growth of cells in BanlanCD and FreeStyle media was slow, and the cells basically stopped growing during the culture of the second passage. Therefore, BanlanCD and FreeStyle media were not suitable for the growth of suspended HEK293 cells, and subsequent experiments were no longer conducted. At this time, the suspended cells cultured in three serum-free media: OPM-293 CD03, LV-MAX and Expi293 were photographed to record the colony morphology. As shown in Fig. 3, the suspended cells in the three media were in good growth status. The suspended cells cultured in three serum-free media were inoculated into cell culture bottles at 0.35 × 10⁶ cells/ml, and cultured for seven days. Cell density and vitality were measured by a cell counter (manufacturer: Beckman, model: Vicell) every day, and the growth curve and vitality curve were drawn. As shown in Fig. 4, the suspended cells in OPM-293 CD03 medium were in good growth status, and the cell density reached approximately 1.3 × 10'' cells/ml on day 7. The cell viability remained above 60% on day 7.

Cells that can reach a higher density were screened out through passaging. Specifically, the cell line with the highest suspension density of 1.0 × 10⁷ cells/ml in OPM-293 CD03 medium was selected and named HEK293 suspension cells. The cells were cryopreserved to a final density of 1.0 × 10⁷ cells/ml, and used as the selected cell line adapted to serum-free suspension culture. During this step, 10-20 cells clustered together.

### Example 2, Monoclonal origin and cell clumping optimization of Serum-free suspension adapted HEK293

In order to further solve the problems about clumping and monoclonal origin of serum-free suspension adapted HEK293 cells, the cells adapted to OPM-293 CD03 medium were taken and subjected to clone screening by two rounds of limiting dilution plus monoclonal imaging, and the specific steps were as follows.

### First round of limiting dilution

After harvesting cells, the cells were diluted to a cell density of 1 × 10⁵ cells/ml. 100 µl of cell suspension with a cell density of 1 × 10⁵ cells/ml was pipetted into 9.9 ml of OPM-293 CD03 cell medium, and the cell density was adjusted to 1 × 10³ cells/ml.

0.5 ml of cell suspension with a cell density of 1 × 10³ cells/ml was pipetted into 100 ml of cell medium, and the cell density was adjusted to 5 cells/ml. The diluted cell suspension was added to each well of the 96-well plate at 100 µl/well.

After 7 days, observation was performed and a single-growing cell was picked out through a microscope. The single-growing cell appeared as one cell clump in the 96-well, while non-single-growing cell appeared as two or more cell clumps.

The medium was replenished every 7 days, and the cell growth rate was accelerated after 14 days.

When the single-growing cell reached 50% confluency, it was transferred to a 24-well plate, 1.5 ml of fresh screening medium was added, and the plate was let stand for culture.

On day 3, observation began. When the confluency of cells in 2/3 wells reached 50%, the cells were transferred to a 6-well plate, and 1 ml of fresh screening medium was added to the 6-well plate which was placed in a shaker at 125 rpm for culture.

If the cells were in poor state, the cells in the 6-well plate were passaged on day 6, and individual clones with higher density were diluted, and then culture was continued until the cell state was restored, and 1 ml of fresh screening medium was added and the plate was placed in a shaker at 125 rpm for culture.

When the confluency of cells in 2/3 wells in the 6-well plate reached 80%, the cells in the 6-well plate were transferred to a 125 ml shake flask for suspension culture. After the shake flask was placed on the shaker, the cells were observed by microscope every day, and the cell growth density and state were examined by microscope, and the clones with fast growth rate, no clumping or light clumping were screened out.

When the density of most cells was higher than 3 × 10⁶cells/ml and the viability was above 95%, transfection could be performed. Specifically, a part of the cells was separated from the shake flask and transfected with the GFP main plasmid AAV2 as follows (the original shake flask continued to be used for passaging). 5 optimal monoclonal cells with high transfection titer and good dispersibility were selected based on cell dispersibility and virus titer.

Specifically, serum-free suspension adapted HEK293 cells and control cell line (Cat#: A35347, Thermo fisher, *i.e.,* the virus producer cells in the LV-MAX^{™} lentivirus production system (Cat#: A35684), referred to as VPC in this article), were inoculated into a 125 ml shake flask at 0.55 × 10⁶ cells /ml and cultured in suspension for three days. The cell suspension was taken from the culture shake flask containing the suspended cells, and the cells were counted, and transfected when the cell density reached 3 × 10⁶ cells/ mL and the cell viability was greater than 95%.

Cells were taken and put in a suitable amount of medium, the transfection density was 3 × 10⁶ cells/mL and the transfection volume was 30 ml. The cell culture flask was placed into an incubator for suspension culture.

Firstly, a transfection complex was prepared. The medium and PEIpro transfection reagent were stored at 2-8°C with no preheating; the transfection reagent needed to be shaken 4-5 times before use to ensure thorough mixing. This operation did not need to be carried out on ice, as long as this operation was carried out as soon as possible after the reagent was taken out of the refrigerator.

The corresponding amount of plasmids was added to the medium. Each plasmid was added according to the mass ratio of carrier plasmid (pAAV-EGFP): packaging plasmid (pRC2/pRC5/pRC8/pRC9): helper plasmid (pHelper) =1 : 3 : 1, and the dosage of plasmids was 0.5 µg/1 × 10⁶ cells, the mixture was gently pipetted for 4-5 times to make the plasmids mixed evenly. The tube was marked as tube 1-DNA. All the vector sequences were from addgene.

90 µl of PEIpro transfection reagent was added to the transfection buffer medium, and the mixture was gently pipetted for 4-5 times to make them mixed evenly. The tube was marked as tube 2-PEIpro.

The transfection buffer in tube 2 was transferred into tube 1, pipetted repeatedly with a pipette for more than 8-10 times, vortexed with a vortexer 10 s as soon as possible, and allowed to stand at room temperature for 15 min.

When transfecting cells, the transfection complex was slowly added into the liquid containing cells to be transfected with shaking the shake flask gently while adding. After the operation was completed, the cell culture flask was placed in the incubator for suspension culture.

The viruses were harvested after 48 h, and titer detection was performed according to the operation steps in the instructions of the AVVpro^{®} Titration Kit (for real-time PCR, TAKARA, Cat. No.: 6233).

After determining 5 optimal monoclonal cells, the packaging test of virus AAV2 of GFP main plasmid was carried out according to the above steps by using the cells in the corresponding shake flask or their passages. Based on the packaging test results, 5 optimal monoclonal cells were selected, namely A7, A26, A52, A62 and G1.

PCB libraries were built for the TOPS and 20 tubes were frozen respectively.

### Second round of limiting dilution

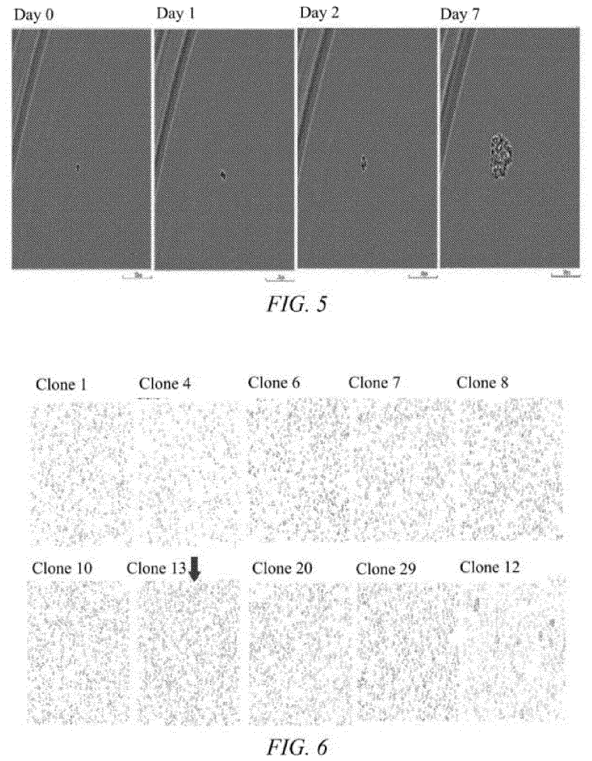

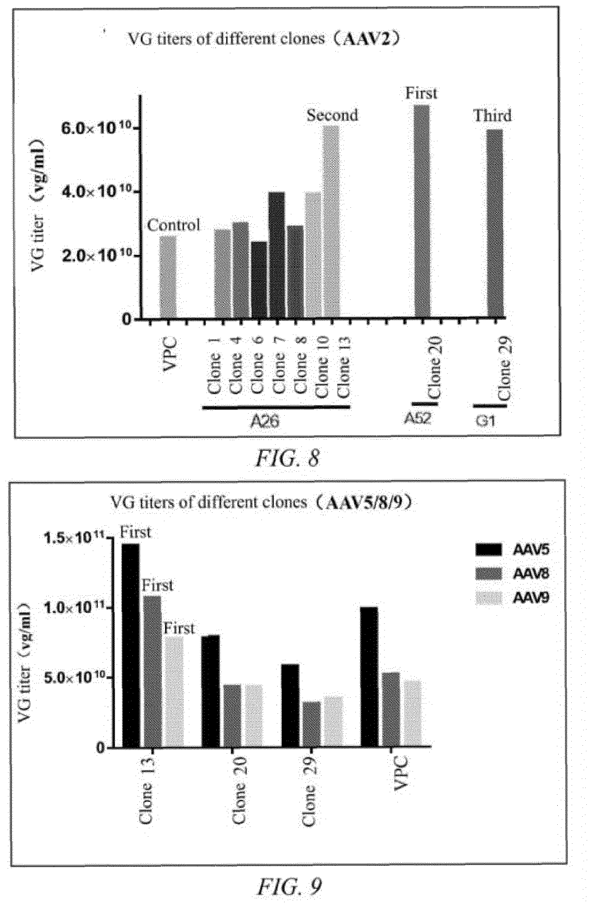

Limiting dilution was performed again on the 5 optimal monoclonal cell clumps determined in the first round, the first round of limiting dilution steps were repeated (that is, repeating the steps of cell dilution until plating), and on the day 0, day 1, day 2 and day 7 of cell growth, monoclonal cells were imaged and photographed with a monoclonal cell strain analyzer (Solentim, Cell Metric), as shown in Fig. 5, and monoclonal cells were screened out. After culture in a shake flask for 3-5 generations, the cells were observed and photographed under microscope. As shown in Fig. 6, clones with no or light clumping were screened out, namely clones 1, 4, 6, 7, 8, 10, 13, 20 and 29, among which clone 13 had the best dispersibility. After the screened clones were passaged and cultured, a part of the cells was separated from the shake flask for transfection with GFP main plasmid AAV2 (the original shake flask continued to be used for passaging). The transfection titer results are shown in Fig. 8. The titers of the screened clones were all higher than those of the control cell line when transfected with AAV2 virus. 3 optimal clones with high transfection titers were selected, namely clones 13, 20 and 29.

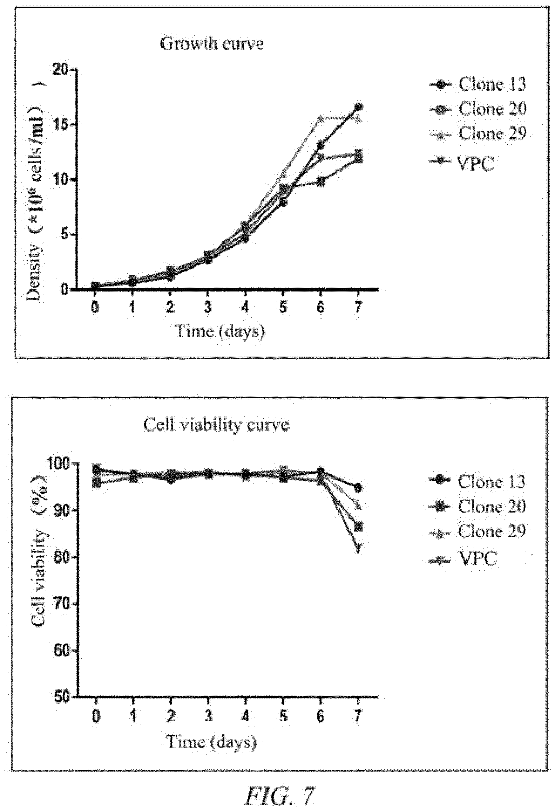

The three monoclonal cells, namely clones 13, 20 and 29, were tested for 7-day growth curve and viability curve in OPM-293 CD03 medium. As shown in Fig. 7 and Table 2, the cells of the three clones were in good growth status. On day 7, the cell density of clone 13 exceeded 1.5 × 10⁷ cells/ml, and the doubling time of clone 13 was the shortest, and the cell viability of all the three cell clones was above 85%. AA5, AAV8, and AAV9 packaging tests were conducted on the cells of the three clones. The results showed that the cells of clone 13 had the highest titers when packaging viruses of these three serotypes, with an average value of approximately 1.0 × 10¹¹ vg/ml, which was significantly higher than that of the control cell line, as shown in Fig. 9.

**Table 2. Doubling time, cell density on day 7 and cell viability on day 7 of the three clones**

| **Clone NO** | **Cell doubling time (h)** | **Cell density on day 7 (10⁶ cells/ml)** | **Cell viability on day 7 (%)** |
|---|---|---|---|
| Clone 13 | 24.3 | 16.6 | 94.9 |
| Clone 20 | 25.3 | 11.9 | 86.6 |
| Clone 29 | 24.8 | 15.6 | 91.2 |
| Control cell line | 24.9 | 12.3 | 81.9 |

### Example 3. Passage stability experiment of clone 13

Cells cultured *in vitro* have a high probability of genetic mutations and epigenetic changes, which may be affected by culture conditions. For example, some types of cells would differentiate when conditions such as cell density, oxygen or carbon dioxide concentration change, leading to the production capacity of these cells decrease as the number of passages increase in the process of producing biological products, and the product quality changes over time and becomes unstable between batches. Therefore, this requires us to verify the stability of such cell strains in biological production technology. We examine the virus production stability of clone 13 through AAV5 virus packaging, and the specific steps are as follows:
Clone 13 was cryopreserved at the 5th, 10th, 15th, and 20th generations during the passaging process. After the 20th generation was frozen, the clones of the frozen 5th, 10th, 15th and 20th generations were incubated in a 37°C water bath for about 1 min. The cells in the cryovials were quickly thawed until there was no ice in the cryovials, and then the cells were inoculated into a 125 ml shake flask at 0.55 × 10⁶ cells/ml and cultured in suspension for three days. The cell suspension was taken from the culture shake flask containing the suspended cells, and the cells were counted, and transfected when the cell density reached 3 × 10⁶ cells/ mL and the cell viability was greater than 95%.

Cells were taken and put in a suitable amount of OPM-293 CD03 medium, the transfection density was 3 × 10⁶ cells/mL and the transfection volume was 30 ml. The cell culture flask was placed into an incubator for suspension culture.

Firstly, a transfection complex was prepared. The OPM-293 CD03 medium and PEIpro transfection reagent were stored at 2-8°C with no preheating; the transfection reagent needed to be shaken 4-5 times before use to ensure thorough mixing. This operation did not need to be carried out on ice, as long as this operation was carried out as soon as possible after the reagent was taken out of the refrigerator.

The corresponding amount of plasmids was added to the medium. Each plasmid was added according to the mass ratio of carrier plasmid (pAAV-EGFP): packaging plasmid (pRC2/pRC5/pRC8/pRC9): helper plasmid (pHelper) =1 : 3 : 1, and the dosage of plasmids was 0.5 µg/1 × 10⁶ cells, the mixture was gently pipetted for 4-5 times to make the plasmids mixed evenly. The tube was marked as tube 1-DNA. All the vector sequences were from addgene.

90 µl of PEIpro transfection reagent was added to the transfection buffer medium, and the mixture was gently pipetted for 4-5 times to make them mixed evenly. The tube was marked as tube 2-PEIpro.

The transfection buffer in tube 2 was transferred into tube 1, pipetted repeatedly with a pipette for more than 8-10 times, vortexed with a vortexer 10 s as soon as possible, and allowed to stand at room temperature for 15 min.

When transfecting cells, the transfection complex was slowly added into the liquid containing cells to be transfected with shaking the shake flask gently while adding. After the operation was completed, the cell culture flask was placed in the incubator for suspension culture.

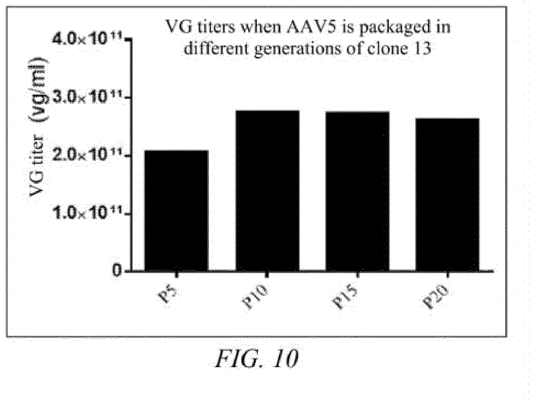

The viruses were harvested after 48 h, and titer detection was performed according to the operation steps in the instructions of the AAVpro^{®} Titration Kit (for real-time PCR, TAKARA, Cat. No.: 6233). The test results were shown in Fig. 10. From Fig. 10, it can be concluded that the ability of clone 13 to produce AAV5 viruses would not decrease with the increase in the number of passages. The ability of clone 13 to package AAV viruses within 20 generations (including the 20th generation) is stable.

In summary, clone 13 was finally determined to be the optimal clone, and the clone 13 cell line was named PowerS-293, which was later deposited in the China General Microbiological Culture Collection Center (CGMCC) with a deposit number of 23020.

The embodiments of the present invention are not limited to the above-mentioned examples, and various modifications and improvements in forms and details may be made to the present invention by those of ordinary skill in the art without departing from the spirit and scope of the present invention, and the modifications and improvements are considered to fall within the scope of protection of the present invention.

## Claims

1. A novel human embryonic kidney HEK293 cell strain, deposited at the China General Microbiological Culture Collection Center with a deposit number of CGMCC NO.: 23020.

2. A method for screening a HEK293 cell strain adapted to serum-free suspension culture, **characterized in that** the method comprises:
i) taking a HEK293 cell adherently cultured in a serum-containing medium, and placing the cell in a serum-free medium for suspension culture, wherein the serum-free medium is selected from OPM-293 CD03 medium, LV-MAX production medium, and Expi293 expression medium, and
ii) selecting a monoclonal cell strain adapted to suspension culture.

3. The method of claim 2, **characterized in that** the serum-containing medium in step i) contains 10% serum.

4. The method of claim 2, **characterized in that** the suspension culture in step i) includes shaking culture at 37°C and 8% CO₂.

5. The method of claim 2, **characterized in that** the suspension culture in step i) includes cell passaging.

6. The method of claim 2, **characterized in that** step ii) comprises selecting a monoclonal cell through at least one round of limiting dilution and photographing and imaging, and culturing the selected monoclonal cell in suspension.

7. The method of claim 6, **characterized in that** culturing the selected cell in suspension in step ii) includes cell passaging.

8. The method of claim 2, **characterized in that** the serum-free medium is OPM-293 CD03 medium.

9. The method of any one of claims 2-8, **characterized in that** the method is carried out without the addition of an anti-clumping agent.

10. A cell strain screened out by using the method of any one of claims 2-9.

11. The cell strain of claim 10, **characterized in that** the cell strain does not clump in a liquid medium.

12. A medium selected from LV-MAX production medium, Expi293 expression medium and OPM-293 CD03 medium for use in screening a HEK293 cell strain adapted to suspension culture.

13. A method for preparing a viral vector using the cell strain of any one of claims 1, 10 and 11, **characterized in that** the method comprises:
i) performing suspension culture on the cell strain;
ii) introducing a viral vector expression system into the cell strain;
iii) culturing the cell strain under a condition favorable for the production of the viral vector; and
iv) collecting the viral vector.

14. The method of claim 13, **characterized in that** after step iv), step v) is further included to determine the titer of the viral vector.

15. The method of claim 13, **characterized in that** the viral vector is an adeno-associated virus vector.

16. The method of claim 15, **characterized in that** the viral vector expression system includes a heterologous nucleic acid-containing carrier plasmid, a rep-cap-containing packaging plasmid, and a helper plasmid.
